# EUROPEAN PATENT APPLICATION

(11) **EP 4 306 511 A1**
(43) Date of publication of application: **17.01.2024**
(21) Application number: 22766394.5
(22) Date of filing: 11.03.2022
(51) Int. Cl.: C07D 213/89, A61K 31/44, A61P 29/00, A61P 11/14

(54) **CRYSTAL FORM OF PYRIDINE NITROGEN OXIDE COMPOUND AND USE THEREOF**

(30) Priority: 11.03.2021 CN 202110265997; 01.03.2022 CN 202210195905
(71) Applicant: Shanghai Jemincare Pharmaceuticals Co., Ltd., Shanghai 201203 (CN); Jiangxi Jemincare Group Co., Ltd., Nanchang, Jiangxi 330000 (CN)
(72) Inventor: ZHANG, Yong, Shanghai 201203 (CN); CAO, Cheng, Shanghai 201203 (CN); WAN, Qingwei, Shanghai 201203 (CN); CHENG, Hongming, Shanghai 201203 (CN); PENG, Jianbiao, Shanghai 201203 (CN)
(74) Representative: J A Kemp LLP
(86) International application number: PCT/CN2022/080430
(87) International publication number: WO 2022/188872

(57) **Abstract**

A crystal form of a pyridine nitrogen oxide compound and the use thereof. Specifically, the present invention relates to a crystal form of a compound of formula (I), a pharmaceutical composition and the use thereof.

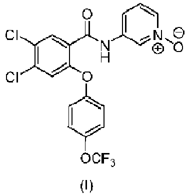

## Description

The present application claims the following rights of priority:
Application number: CN 202110265997.3, application date: March 11, 2021;
Application number: CN 202210195905.3, application date: March 1, 2022.

### TECHNICAL FIELD

The present disclosure relates to a crystal form of a compound represented by formula (I), a pharmaceutical composition, and a use as a voltage-gated sodium channel (NaV) blocker thereof.

### BACKGROUND ART

Pain is one of the most common clinical symptoms and is the fifth vital sign after respiration, pulse, blood pressure and body temperature, and seriously affects the quality of life of patients. According to statistics, the global analgesic market was approximately US$36 billion in 2018 and is expected to reach US$56 billion in 2023. Acute moderate and severe patients mainly depend on opioids, accounting for about two-thirds of the analgesic market share, which will grow steadily at a compound annual growth rate of 2.5% in the future. The number of chronic pain patients, mainly those suffering from neuropathic pain and arthritis pain, is increasing year by year, and the market is expected to show a compound annual growth rate of about 18%, which will be the main driving force for the continued growth of the global pain market over the next decade.

Neuropathic pain is a chronic pain caused by damage or disease of the peripheral somatosensory nervous system, and its symptoms include spontaneous pain and allodynia to normal harmless stimulation. Common etiology of neuropathic pain includes diabetes, herpes zoster, spinal cord injury, apoplexy, multiple sclerosis, cancer, HIV infection, lumbar or cervical radiculopathy, and trauma or post-operative nerve damage. Osteoarthritis, also known as degenerative arthritis, is the degeneration of bone and articular cartilage caused by a variety of factors, which can lead to uneven surfaces of joint bone and may form bone spurs, and the clinical manifestations are mainly joint pain and joint stiffness. Long-term pain not only affects the patient's ability to sleep, work and live, but also increases the incidence of emotional disorders such as depression or anxiety, thus bringing a heavy economic burden to the patient's family and society.

According to the data released by the Neuropathic Pain Special Interest Group (NeuPSIG), the prevalence of neuropathic pain is about 3.3% to 8.2%. According to this calculation, there are at least 50 million patients in China alone. In 2017, there were 30.5 million patients with neuropathic pain in the US, Japan and the five major markets of the EU (France, Germany, Italy, Spain and the UK), and the trend is increasing year by year. Neuropathic pain is one of the most difficult diseases to treat, and most of the current treatment options are still unsatisfactory. It has been reported that only 14.9% of outpatients can attain timely pain relief through drug treatment; that is, about 85% of pain patients do not receive timely and effective drug treatment, so some patients have to seek treatment by surgical intervention. At present, the first-line drugs used in the clinical treatment of neuropathic pain are mainly calcium channel modulators (such as pregabalin, gabapentin), tricyclic antidepressants and 5-hydroxytryptamine, norepinephrine reuptake inhibitors (such as duloxetine, venlafaxine and other anticonvulsants and antidepressants). These drugs have limited efficacy and are associated with various adverse reactions. Duloxetine is one of the first-line drugs for the treatment of neuropathic pain, and the main side effects include gastrointestinal reaction, nausea, drowsiness, dry mouth, sweating and dizziness, resulting in a withdrawal rate of 15% to 20%. Gabapentin and pregabalin of the antiepileptic drugs are the main drugs for the treatment of neuropathic pain, causing many other adverse effects of dizziness, somnolence, peripheral edema, weight gain, weakness, headache and dry mouth. In recent years, pregabalin has also been found to cause drug use-related suicidal ideation and self-injury behavior in a very small number of patients.

The number of patients with osteoarthritis is enormous, it is estimated that there are more than 400 million patients with osteoarthritis in the world, and the number of patients in China exceeds 100 million. There is currently no effective treatment for osteoarthritis pain. Clinically available treatments include physical therapy, drug therapy and surgery. Physical therapy includes hyperthermia, hydrotherapy, ultrasound and massage, etc. as well as assistive devices to reduce joint pressure and relieve pain, but the effect is limited, and most patients still need to depend on drugs for treatment. These drugs all have different degrees of side effects. Nonsteroidal anti-inflammatory drugs are only suitable for mild to moderate pain and have gastrointestinal side effects and cardiovascular and cerebrovascular risks. Opioid analgesics are used for severe pain but have significant side effects such as nausea and vomiting, constipation, and drug dependence, and are not suitable for long-term use. Therefore, it is of great economic and social importance to develop new mechanisms for targeting new targets as well as safe and effective analgesic drugs to meet unmet clinical needs.

Recent research results have gradually revealed that sodium channel subtype 1.8 (NaV1.8) plays an important role in the occurrence and transmission of pain sensation. NaV1.8 is a voltage-gated sodium channel, which is mainly expressed in afferent neurons including sensory neurons and plays an important role in maintaining the excitability of nociceptive sensory neurons, the release and persistence of action potentials, and the regulation of pain sensitivity by controlling sodium ions into and out of cells. Patients with NaV1.8 activating mutations develop paroxysmal pain due to small-fiber neuropathy (the fiber damage of Aδ fibers and unmyelinated C-fiber primarily responsible for pain transmission). Diseases such as chronic inflammation and diabetes can cause increased expression or altered properties of NaV1.8, which sensitize nociceptive neurons, causing a variety of pain. However, NaV1.8 knockout mice are insensitive to pain.

With the determination of the status of NaV1. 8 in chronic pain, drug research based on this target is also increasingly popular. At present, internationally there is a small molecule blocker in clinical phase II, and many other small molecule blockers and antibodies are in preclinical development, while there are no other new drugs in research and development for this target in China. At the forefront of the research and development is the US company Vertex's small molecule NaV1.8 blocker VX-150, which has been tested in a phase II clinical trial in patients with osteoarthritis, acute pain and pain caused by small-fiber neuropathy, with all three studies producing positive results, indicating that inhibiting the activity of NaV1.8 can alleviate a variety of pain, including neuropathic pain. At present, VX-150 has obtained the designation of breakthrough therapy by the FDA in the US for the treatment of moderate to severe pain, once again proving that NaV1.8 is a potential target for analgesia. In addition, the mechanism and the phase II clinical trials of NaV1.8 blockers show that they have a wide range of applications, including neuropathic pain, osteoarthritis pain, acute injury pain and other pain. Furthermore, it has relatively high safety, does not cause addiction, and does not have the gastrointestinal side effects and cardiovascular and cerebrovascular side effects of nonsteroidal anti-inflammatory drugs; it can also be used in combination with other analgesics to enhance efficacy and reduce side effects.

In recent years, studies have also shown that sodium channel subtype 1.8 (NaV1.8) has a certain regulatory effect on cough, and NaV1.8 blockers may be used as potential drugs for the treatment of cough.

The application No. PCT/CN2020/114700 (application date of September 11, 2020) provides a NaV1.8 blocker, the structure being as follows:

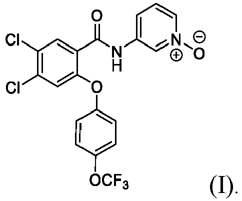

### SUMMARY OF THE INVENTION

In one aspect of the present disclosure, the present disclosure provides a crystal form A of a compound represented by formula (I), the X-ray powder diffraction pattern thereof comprises characteristic diffraction peaks at the following angles of 2θ: 16.63±0.2°, 18.04±0.2°, 20.59±0.2°, 23.38±0.2°, 23.96±0.2°, 29.19±0.2°.

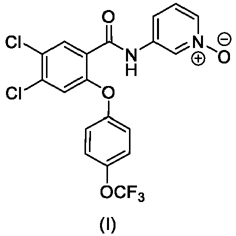

In some embodiments of the present invention, the X-ray powder diffraction pattern of the crystal form A comprises characteristic diffraction peaks at the following angles of 2θ: 12.46±0.2°, 13.11±0.2°, 16.63±0.2°, 18.04±0.2°, 20.59±0.2 °, 23.38±0.2°, 23.96±0.2°, 27.66±0.2°, 29.19±0.2° and 29.82±0.2°.

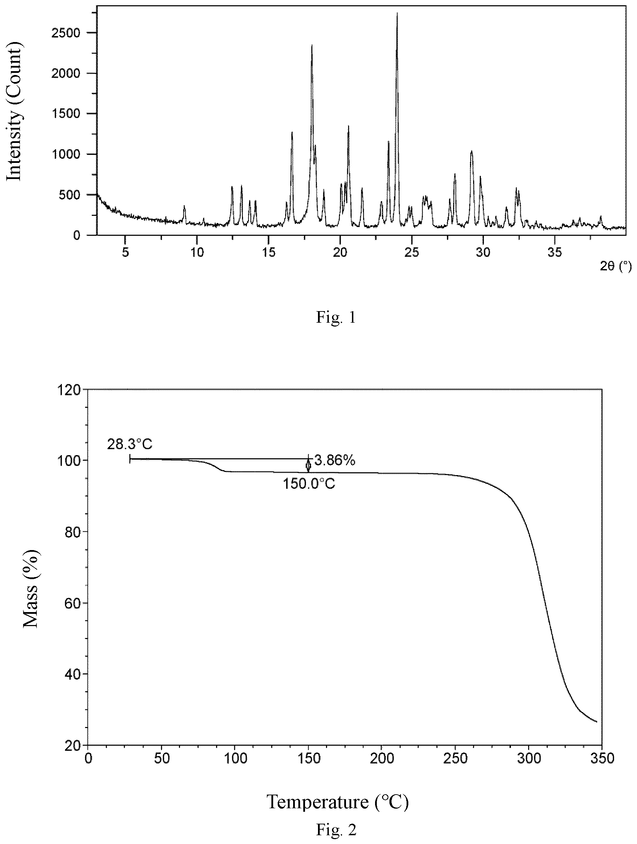

In some embodiments of the present disclosure, the X-ray powder diffraction pattern of the crystal form A has an X-ray powder diffraction pattern, substantially as shown in Fig. 1.

In some embodiments of the present disclosure, the analytical data of the X-ray powder diffraction pattern of the crystal form A is as shown in Table 1.

**Table 1**

| 2θ (degrees) | Peak height (count) | Left half-peak width (2θ, degrees) | Interplanar spacing (Å) | Relative intensity (%) |
|---|---|---|---|---|
| 9.12 | 194.66 | 0.1023 | 9.69 | 7.34 |
| 10.45 | 62.68 | 0.0768 | 8.46 | 2.36 |
| 12.46 | 474.47 | 0.1279 | 7.10 | 17.89 |
| 13.11 | 484.66 | 0.0768 | 6.75 | 18.28 |
| 13.69 | 313.40 | 0.0768 | 6.47 | 11.82 |
| 14.08 | 307.40 | 0.1023 | 6.29 | 11.59 |
| 16.26 | 297.26 | 0.0768 | 5.45 | 11.21 |
| 16.63 | 1162.91 | 0.1023 | 5.33 | 43.85 |
| 18.04 | 2179.55 | 0.1023 | 4.92 | 82.19 |
| 18.27 | 999.88 | 0.0768 | 4.86 | 37.70 |
| 18.87 | 459.70 | 0.1023 | 4.70 | 17.33 |
| 20.09 | 530.66 | 0.1023 | 4.42 | 20.01 |
| 20.37 | 538.27 | 0.0768 | 4.36 | 20.30 |
| 20.59 | 1214.61 | 0.1023 | 4.31 | 45.80 |
| 21.55 | 480.61 | 0.1279 | 4.12 | 18.12 |
| 22.90 | 315.04 | 0.1023 | 3.88 | 11.88 |
| 23.38 | 1059.72 | 0.1279 | 3.81 | 39.96 |
| 23.96 | 2651.91 | 0.1535 | 3.71 | 100.00 |
| 24.80 | 255.01 | 0.0768 | 3.59 | 9.62 |
| 24.98 | 236.50 | 0.1023 | 3.56 | 8.92 |
| 25.81 | 373.11 | 0.1023 | 3.45 | 14.07 |
| 26.01 | 351.26 | 0.1535 | 3.43 | 13.25 |
| 26.35 | 323.75 | 0.1023 | 3.38 | 12.21 |
| 27.66 | 351.59 | 0.1279 | 3.22 | 13.26 |
| 28.01 | 659.34 | 0.1279 | 3.19 | 24.86 |
| 29.19 | 952.09 | 0.1560 | 3.06 | 35.90 |
| 29.29 | 790.62 | 0.0936 | 3.05 | 29.81 |
| 29.82 | 596.15 | 0.1560 | 2.99 | 22.48 |
| 30.37 | 119.63 | 0.1560 | 2.94 | 4.51 |
| 30.91 | 137.38 | 0.1560 | 2.89 | 5.18 |
| 31.64 | 253.56 | 0.1560 | 2.83 | 9.56 |
| 32.30 | 492.61 | 0.1248 | 2.77 | 18.58 |
| 32.49 | 440.62 | 0.1872 | 2.75 | 16.62 |
| 33.01 | 87.51 | 0.2496 | 2.71 | 3.30 |
| 33.68 | 59.86 | 0.1560 | 2.66 | 2.26 |
| 34.00 | 46.50 | 0.1248 | 2.63 | 1.75 |
| 35.68 | 39.26 | 0.3744 | 2.51 | 1.48 |
| 36.30 | 87.36 | 0.1872 | 2.47 | 3.29 |
| 36.74 | 112.34 | 0.1872 | 2.44 | 4.24 |
| 37.05 | 51.08 | 0.1872 | 2.42 | 1.93 |
| 38.22 | 143.30 | 0.0936 | 2.35 | 5.40 |

In some embodiments of the present disclosure, when the crystal form A is subjected to thermogravimetric analysis (TGA), the crystal form A heated to 150°C has a weight loss of 3.9%, with an error tolerance of±0.2%.

In some embodiments of the present disclosure, the crystal form A has a thermogravimetric analysis substantially as shown in Fig. 2.

In some embodiments of the present disclosure, the differential scanning calorimetry (DSC) pattern of the crystal form A has an endothermic peak at 101.1°C±3°C.

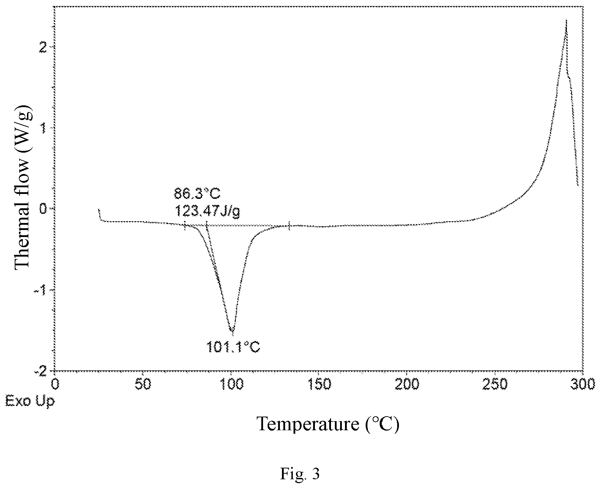

In some embodiments of the present disclosure, the crystal form A has a DSC differential scanning calorimetry curve substantially as shown in Fig. 3.

In some embodiments of the present disclosure, the crystal form A is a hydrate, and the moisture content of the hydrate is 2.0wt% to 6.0wt%, with an error tolerance of ±0.2%.

In some embodiments of the present disclosure, the crystal form A is a hydrate, and the moisture content of the hydrate is 3.0wt% to 5.0wt%, with an error tolerance of ±0.2%.

In some embodiments of the present disclosure, the crystal form A is a hydrate, and the moisture content of the hydrate is 2.1wt%, 2.3wt%, 2.5wt%, 2.8wt%, 3.1wt%, 3.2wt%, 3.5wt%, 3.8wt%, 4.1wt%, 4.2wt%, 4.5wt%, 4.8wt%, 5.1wt%, 5.2wt%, 5.5wt%, 5.8wt%, or 6.1wt%, with an error tolerance of ±0.2%.

In one aspect of the present disclosure, the present disclosure provides a crystal form B of the compound represented by formula (I), the X-ray powder diffraction pattern thereof comprises characteristic diffraction peaks at the following angles of 2θ: 12.28±0.2°, 14.47±0.2°, 18.86± 0.2°, 23.09±0.2°, 25.50±0.2° and 27.58±0.2°.

In some embodiments of the present disclosure, the X-ray powder diffraction pattern of the crystal form B comprises characteristic diffraction peaks at the following angles of 2θ: 12.28±0.2°, 14.47±0.2°, 16.81±0.2°, 18.86±0.2°, 19.78±0.2 °, 23.09±0.2°, 25.09±0.2°, 25.50±0.2°, 27.58±0.2° and 28.19±0.2°.

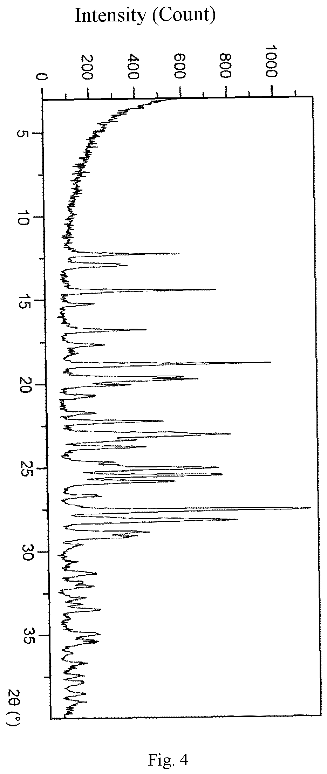

In some embodiments of the present disclosure, the X-ray powder diffraction pattern of the crystal form B has an X-ray powder diffraction pattern substantially as shown in Fig. 4.

In some embodiments of the present disclosure, the analytical data of the X-ray powder diffraction pattern of the crystal form B is as shown in Table 2.

**Table 2**

| 2θ (degrees) | Peak height (count) | Left half-peak width (2θ, degrees) | Interplanar spacing (Å) | Relative intensity (%) |
|---|---|---|---|---|
| 12.28 | 472.40 | 0.1023 | 7.21 | 44.21 |
| 12.86 | 233.62 | 0.1023 | 6.88 | 21.86 |
| 12.98 | 243.81 | 0.0768 | 6.82 | 22.82 |
| 14.47 | 616.06 | 0.0768 | 6.12 | 57.65 |
| 15.26 | 130.53 | 0.1279 | 5.81 | 12.21 |
| 16.81 | 333.57 | 0.1279 | 5.27 | 31.22 |
| 17.68 | 178.98 | 0.1279 | 5.02 | 16.75 |
| 18.86 | 858.34 | 0.1023 | 4.70 | 80.33 |
| 19.62 | 491.77 | 0.0768 | 4.53 | 46.02 |
| 19.78 | 565.46 | 0.1279 | 4.49 | 52.92 |
| 20.08 | 287.82 | 0.1279 | 4.42 | 26.93 |
| 20.74 | 122.89 | 0.1023 | 4.28 | 11.50 |
| 21.75 | 144.28 | 0.1535 | 4.09 | 13.50 |
| 22.27 | 422.29 | 0.1535 | 3.99 | 39.52 |
| 22.94 | 460.81 | 0.0768 | 3.88 | 43.12 |
| 23.09 | 725.40 | 0.1535 | 3.85 | 67.88 |
| 23.37 | 317.93 | 0.1279 | 3.81 | 29.75 |
| 23.80 | 361.16 | 0.1279 | 3.74 | 33.80 |
| 24.70 | 219.92 | 0.0768 | 3.60 | 20.58 |
| 25.09 | 664.34 | 0.1535 | 3.55 | 62.17 |
| 25.50 | 689.54 | 0.1791 | 3.49 | 64.53 |
| 25.87 | 488.02 | 0.1279 | 3.44 | 45.67 |
| 26.68 | 147.73 | 0.1535 | 3.34 | 13.82 |
| 27.58 | 1068.58 | 0.1791 | 3.23 | 100.00 |
| 28.19 | 733.98 | 0.1791 | 3.17 | 68.69 |
| 28.89 | 349.01 | 0.1535 | 3.09 | 32.66 |
| 29.15 | 298.10 | 0.1791 | 3.06 | 27.90 |
| 29.64 | 67.14 | 0.1535 | 3.01 | 6.28 |
| 30.69 | 32.97 | 0.3070 | 2.91 | 3.09 |
| 31.34 | 133.89 | 0.2047 | 2.85 | 12.53 |
| 32.10 | 115.56 | 0.1023 | 2.79 | 10.81 |
| 32.80 | 90.71 | 0.1023 | 2.73 | 8.49 |
| 33.49 | 156.59 | 0.1791 | 2.68 | 14.65 |
| 34.96 | 149.08 | 0.2047 | 2.57 | 13.95 |
| 35.41 | 127.35 | 0.2558 | 2.54 | 11.92 |
| 36.72 | 69.79 | 0.1535 | 2.45 | 6.53 |
| 37.44 | 62.41 | 0.1535 | 2.40 | 5.84 |
| 37.85 | 81.06 | 0.2047 | 2.38 | 7.59 |
| 38.52 | 89.49 | 0.2047 | 2.34 | 8.37 |
| 39.01 | 78.00 | 0.2047 | 2.31 | 7.30 |

In some embodiments of the present disclosure, when the crystal form B is subjected to thermogravimetric analysis (TGA), the crystal form B heated to 150°C has a weight loss of 1.2%, with an error tolerance of±0.1%.

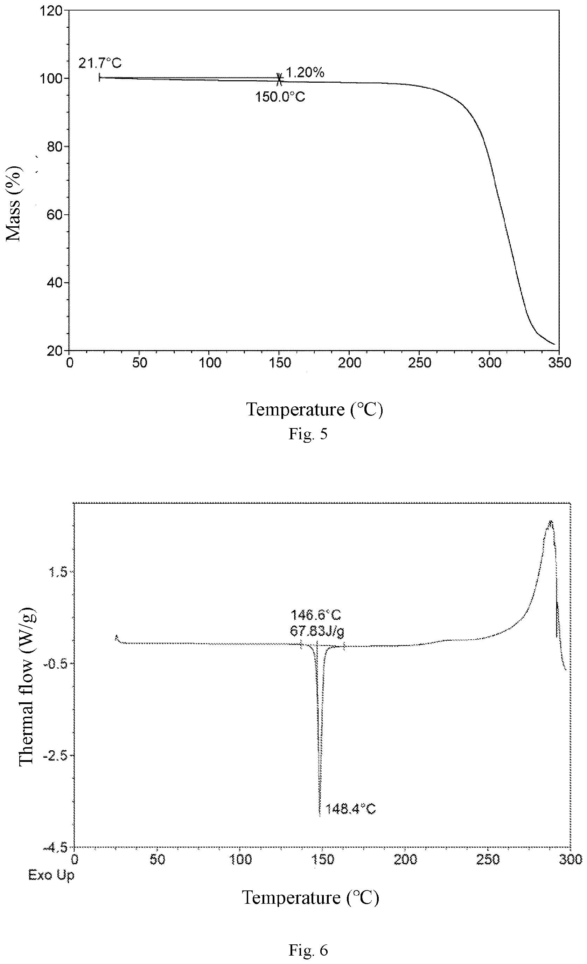

In some embodiments of the present disclosure, the crystal form B has a thermogravimetric analysis substantially as shown in Fig. 5.

In some embodiments of the present disclosure, the differential scanning calorimetry (DSC) pattern of the crystal form B has an endothermic peak at 148.4°C±3°C.

In some embodiments of the present disclosure, the crystal form B has a DSC differential scanning calorimetry curve substantially as shown in Fig. 6.

In some embodiments of the present disclosure, the crystal form B is an anhydrous crystal form.

In one aspect of the present disclosure, the present disclosure provides a crystal form C of the compound represented by formula (I), the X-ray powder diffraction pattern thereof comprises characteristic diffraction peaks at the following angles of 2θ: 8.22±0.2°, 17.33±0.2°, 19.55±0.2°, 20.27±0.2°, 21.99±0.2° and 24.90±0.2°.

In some embodiments of the present disclosure, the X-ray powder diffraction pattern of the crystal form C comprises characteristic diffraction peaks at the following angles of 2θ: 8.22±0.2°, 13.80±0.2°, 17.33±0.2°, 19.55±0.2°, 20.27±0.2 °, 21.99±0.2°, 23.00±0.2°, 23.95±0.2°, 24.90±0.2° and 26.10±0.2°.

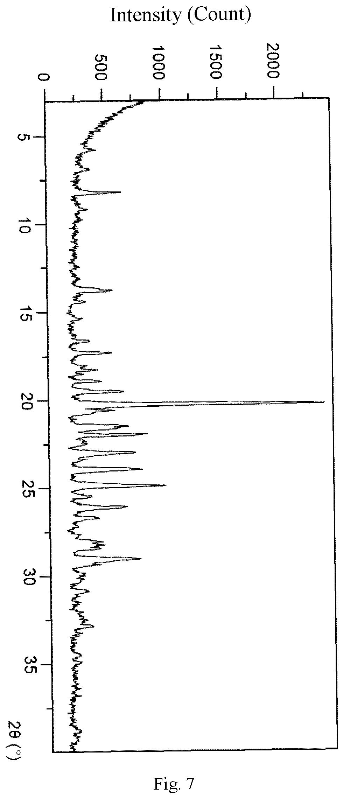

In some embodiments of the present disclosure, the X-ray powder diffraction pattern of the crystal form C has an X-ray powder diffraction pattern substantially as shown in Fig. 7.

In some embodiments of the present disclosure, the analytical data of the X-ray powder diffraction pattern of the crystal form C is as shown in Table 3.

**Table 3**

| 2θ (degrees) | Peak height (count) | Left half-peak width (2θ, degrees) | Interplanar spacing (Å) | Relative intensity (%) |
|---|---|---|---|---|
| 5.79 | 83.95 | 0.1535 | 15.26 | 3.81 |
| 6.91 | 96.81 | 0.2047 | 12.80 | 4.40 |
| 8.22 | 393.33 | 0.0768 | 10.75 | 17.87 |
| 9.15 | 66.45 | 0.3070 | 9.67 | 3.02 |
| 13.80 | 345.63 | 0.1023 | 6.42 | 15.71 |
| 14.40 | 124.15 | 0.1279 | 6.15 | 5.64 |
| 15.36 | 98.48 | 0.1535 | 5.77 | 4.47 |
| 16.66 | 163.54 | 0.1279 | 5.32 | 7.43 |
| 17.33 | 360.24 | 0.1279 | 5.12 | 16.37 |
| 18.30 | 236.07 | 0.1023 | 4.85 | 10.73 |
| 18.95 | 269.47 | 0.1279 | 4.68 | 12.25 |
| 19.55 | 463.42 | 0.1279 | 4.54 | 21.06 |
| 20.27 | 2200.59 | 0.1535 | 4.38 | 100.00 |
| 20.59 | 369.74 | 0.0768 | 4.31 | 16.80 |
| 21.48 | 509.04 | 0.1279 | 4.14 | 23.13 |
| 21.99 | 614.82 | 0.1279 | 4.04 | 27.94 |
| 22.46 | 118.99 | 0.2558 | 3.96 | 5.41 |
| 23.00 | 566.12 | 0.1279 | 3.87 | 25.73 |
| 23.95 | 625.19 | 0.2303 | 3.71 | 28.41 |
| 24.90 | 800.88 | 0.1535 | 3.58 | 36.39 |
| 25.49 | 180.75 | 0.1279 | 3.49 | 8.21 |
| 26.10 | 469.25 | 0.1791 | 3.41 | 21.32 |
| 26.70 | 231.05 | 0.1535 | 3.34 | 10.50 |
| 28.06 | 247.13 | 0.2047 | 3.18 | 11.23 |
| 28.44 | 251.73 | 0.1023 | 3.14 | 11.44 |
| 29.04 | 559.88 | 0.1535 | 3.08 | 25.44 |
| 30.02 | 78.93 | 0.5117 | 2.98 | 3.59 |
| 30.88 | 133.85 | 0.1535 | 2.90 | 6.08 |
| 32.85 | 182.12 | 0.1535 | 2.73 | 8.28 |

In some embodiments of the present disclosure, when the crystal form C is subjected to thermogravimetric analysis (TGA), the crystal form C heated to 80°C has a weight loss of 1.2%, and the crystal form C heated from 80°C to 150°C has a step weight loss of 7.0%, with an error tolerance of±0.1%.

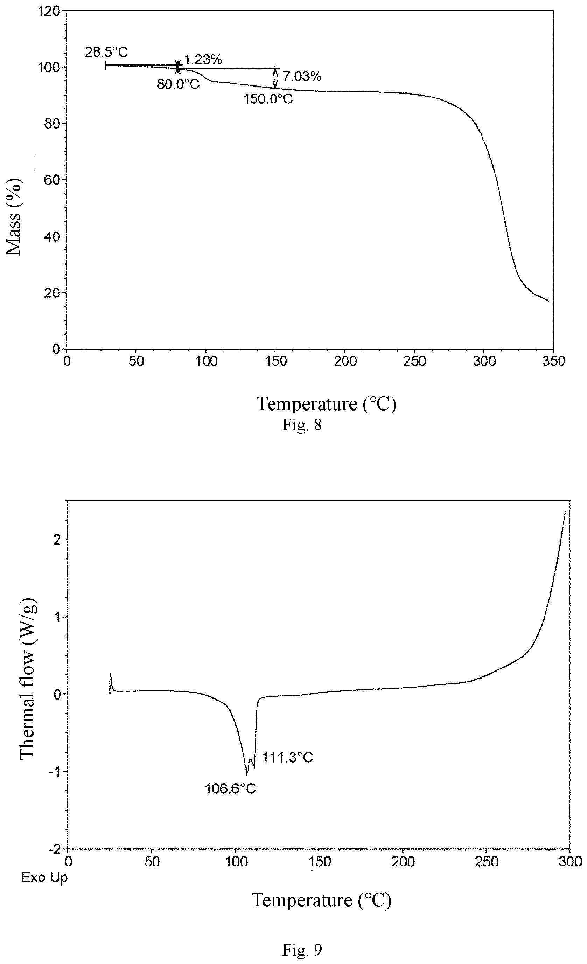

In some embodiments of the present disclosure, the crystal form C has a thermogravimetric analysis substantially as shown in Fig. 8.

In some embodiments of the present disclosure, the differential scanning calorimetry (DSC) pattern of the crystal form C has an overlapping endothermic peak at 106.6±3°C and 111.3±3°C.

In some embodiments of the present disclosure, the crystal form C has a DSC differential scanning calorimetry curve substantially as shown in Fig. 9.

In some embodiments of the present disclosure, the crystal form C is a 1,4-dioxane solvate, and the content of the 1,4-dioxane is 3wt% to 17wt%, with an error tolerance of ±0.2%.

In some embodiments of the present disclosure, the crystal form C is a 1,4-dioxane solvate, and the content of the 1,4-dioxane is 6wt% to 16wt%, with an error tolerance of ±0.2%.

In some embodiments of the present disclosure, the crystal form C is a 1,4-dioxane solvate, and the content of the 1,4-dioxane is 3.1wt %, 3.3wt%, 3.5wt%, 3.8wt%, 4.1wt%, 4.3wt%, 4.5wt%, 4.8wt%, 5.1wt%, 5.3wt%, 5.5wt%, 5.8wt%, 6.1wt%, 6.3wt%, 6.5wt%, 6.8wt%, 7.1wt%, 7.3wt%, 7.5wt%, 7.8wt%, 8.1wt%, 8.3wt%, 8.5wt%, 8.8wt%, 9.1wt%, 9.3wt%, 9.5wt%, 9.7wt%, 10.1wt%, 10.3wt%, 10.5wt%, 10.8wt%, 11.1wt%, 11.3wt%, 11.5wt%, 11.8wt%, 12.1wt%, 12.3wt%, 12.5wt%, 12.8wt%, 13.1wt%, 13.3wt%, 13.5wt%, 13.8wt%, 14.1wt%, 14.3wt%, 14.5wt%, 14.8wt%, 15.1wt%, 15.3wt%, 15.5wt%, 15.8wt%, 16.1wt%, 16.3wt%, 16.5wt%, 16.8wt%, or 17.1wt%, with an error tolerance of ±0.02%.

In one aspect of the present disclosure, the present disclosure provides a crystal form D of the compound represented by formula (I), the X-ray powder diffraction pattern thereof comprises characteristic diffraction peaks at the following angles of 2θ: 5.63±0.2°, 16.81±0.2°, 20.40±0.2°, 21.50±0.2°, 22.23±0.2° and 26.08±0.2°.

In some embodiments of the present disclosure, the X-ray powder diffraction pattern of the crystal form D comprises characteristic diffraction peaks at the following angles of 2θ: 5.63±0.2°, 11.02±0.2°, 16.81±0.2°, 19.58±0.2°, 20.40±0.2 °, 21.50±0.2°, 22.23±0.2°, 24.17±0.2°, 26.08±0.2° and 28.44±0.2°.

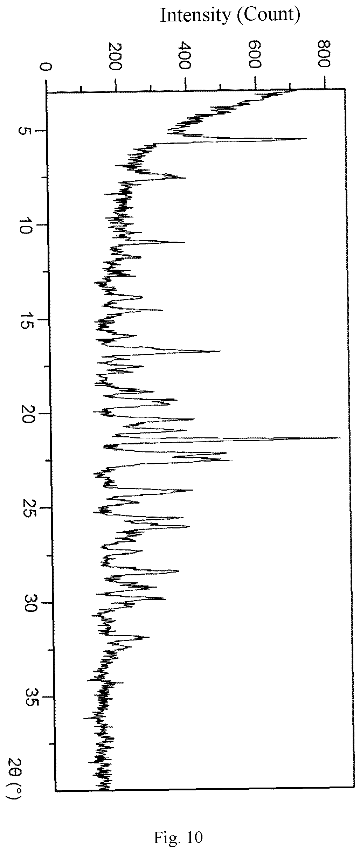

In some embodiments of the present disclosure, the X-ray powder diffraction pattern of the crystal form D has an X-ray powder diffraction pattern substantially as shown in Fig. 10.

In some embodiments of the present disclosure, the analytical data of the X-ray powder diffraction pattern of the crystal form D is as shown in Table 4.

**Table 4**

| 2θ (degrees) | Peak height (count) | Left half-peak width (2θ, degrees) | Interplanar spacing (Å) | Relative intensity (%) |
|---|---|---|---|---|
| 5.63 | 413.93 | 0.1535 | 15.71 | 71.28 |
| 7.55 | 124.98 | 0.2558 | 11.71 | 21.52 |
| 11.02 | 198.60 | 0.1279 | 8.03 | 34.20 |
| 11.78 | 72.39 | 0.1535 | 7.52 | 12.47 |
| 12.70 | 34.66 | 0.1535 | 6.97 | 5.97 |
| 13.90 | 99.56 | 0.2047 | 6.37 | 17.14 |
| 14.60 | 164.05 | 0.0768 | 6.07 | 28.25 |
| 15.94 | 82.72 | 0.1535 | 5.56 | 14.24 |
| 16.81 | 329.74 | 0.1535 | 5.27 | 56.78 |
| 17.57 | 113.52 | 0.1023 | 5.05 | 19.55 |
| 17.85 | 80.20 | 0.0768 | 4.97 | 13.81 |
| 18.91 | 141.15 | 0.0768 | 4.69 | 24.31 |
| 19.33 | 180.52 | 0.0768 | 4.59 | 31.08 |
| 19.58 | 191.08 | 0.1791 | 4.53 | 32.90 |
| 20.40 | 250.42 | 0.1279 | 4.35 | 43.12 |
| 21.00 | 220.24 | 0.1535 | 4.23 | 37.92 |
| 21.50 | 580.73 | 0.1535 | 4.13 | 100.00 |
| 22.23 | 349.48 | 0.1023 | 4.00 | 60.18 |
| 22.57 | 323.98 | 0.1279 | 3.94 | 55.79 |
| 24.17 | 227.60 | 0.1279 | 3.68 | 39.19 |
| 24.75 | 84.42 | 0.1535 | 3.60 | 14.54 |
| 25.62 | 215.20 | 0.1279 | 3.48 | 37.06 |
| 26.08 | 248.26 | 0.1023 | 3.42 | 42.75 |
| 26.77 | 88.81 | 0.1535 | 3.33 | 15.29 |
| 27.36 | 98.02 | 0.1535 | 3.26 | 16.88 |
| 28.44 | 216.72 | 0.1279 | 3.14 | 37.32 |
| 29.28 | 119.82 | 0.2047 | 3.05 | 20.63 |
| 29.86 | 138.38 | 0.1791 | 2.99 | 23.83 |
| 31.94 | 108.97 | 0.2047 | 2.80 | 18.76 |
| 32.43 | 71.24 | 0.1535 | 2.76 | 12.27 |

In some embodiments of the present disclosure, when the crystal form D is subjected to thermogravimetric analysis (TGA), the crystal form D heated to 80°C has a weight loss of 0.9%, and the crystal form D heated from 80°C to 150°C has a weight loss of 7.0%, with an error tolerance of±0.1%.

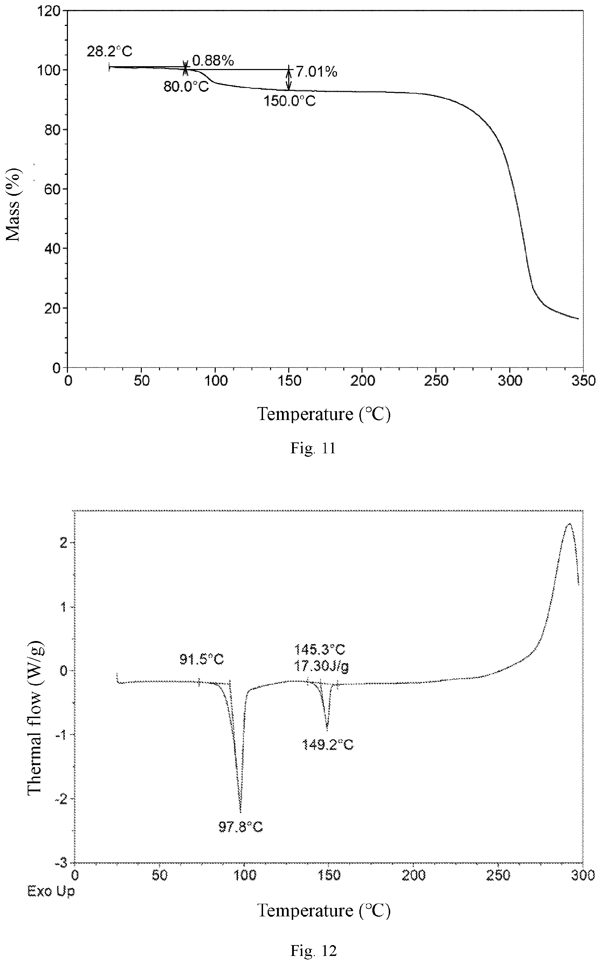

In some embodiments of the present disclosure, the crystal form D has a thermogravimetric analysis substantially as shown in Fig. 11.

In some embodiments of the present disclosure, the differential scanning calorimetry (DSC) pattern of the crystal form D has two endothermic peaks at 97.8±3°C and 149.2±3°C.

In some embodiments of the present disclosure, the crystal form D has a DSC differential scanning calorimetry curve substantially as shown in Fig. 12.

In some embodiments of the present disclosure, the crystal form D is a methyl ethyl ketone solvate, and the content of the methyl ethyl ketone is 4wt% to 14wt%, with an error tolerance of ±0.2%.

In some embodiments of the present disclosure, the crystal form D is a methyl ethyl ketone solvate, and the content of the methyl ethyl ketone is 6wt% to 14wt%, with an error tolerance of ±0.2%.

In some embodiments of the present disclosure, the crystal form D is a methyl ethyl ketone solvate, and the content of the methyl ethyl ketone is 4.1wt%, 4.3wt%, 4.5wt%, 4.8wt%, 5.1wt%, 5.3wt%, 5.5wt%, 5.8wt%, 6.1wt%, 6.3wt%, 6.5wt%, 6.8wt%, 7.1wt%, 7.3wt%, 7.4wt%, 7.5wt%, 7.8wt%, 8.1wt%, 8.3wt%, 8.5wt%, 8.8wt%, 9.1wt%, 9.3wt%, 9.5wt%, 9.8wt%, 10.1wt%, 10.3wt%, 10.5wt%, 10.7wt%, 10.9wt%, 11.1wt%, 11.3wt%, 11.5wt%, 11.8wt%, 12.1wt%, 12.3wt%, 12.5wt%, 12.8wt%, 13.1wt%, 13.3wt%, 13.5wt%, 13.6wt%, 13.8wt% or 14.1wt%, with an error tolerance of ±0.2%.

In one aspect of the present disclosure, the present disclosure provides a crystal form E of the compound represented by formula (I), the X-ray powder diffraction pattern thereof comprises characteristic diffraction peaks at the following angles of 2θ: 5.75±0.2°, 13.71±0.2°, 18.29±0.2°, 20.18±0.2°, 22.92±0.2° and 23.96±0.2°.

In some embodiments of the present disclosure, the X-ray powder diffraction pattern of the crystal form E comprises characteristic diffraction peaks at the following angles of 2θ: 5.75±0.2°, 13.71±0.2°, 16.65±0.2°, 17.17±0.2°, 18.29±0.2 °, 20.18±0.2°, 22.92±0.2°, 23.96±0.2°, 24.76±0.2° and 29.18±0.2°.

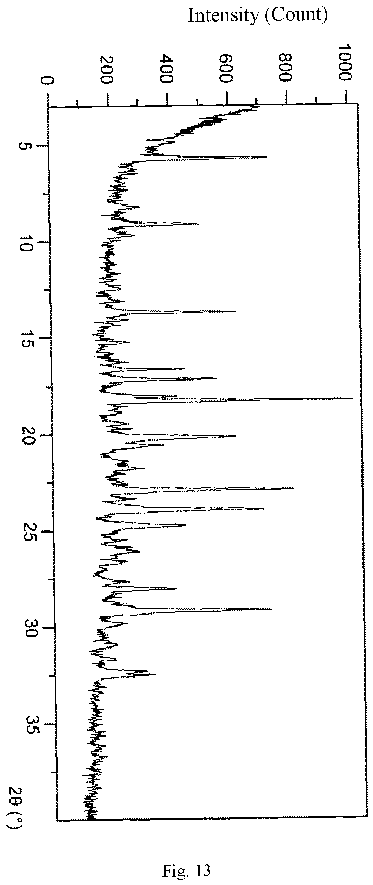

In some embodiments of the present disclosure, the X-ray powder diffraction pattern of the crystal form E has an X-ray powder diffraction pattern substantially as shown in Fig. 13.

In some embodiments of the present disclosure, the analytical data of the X-ray powder diffraction pattern of the crystal form E is as shown in Table 5.

**Table 5**

| 2θ (degrees) | Peak height (count) | Left half-peak width (2θ, degrees) | Interplanar spacing (Å) | Relative intensity (%) |
|---|---|---|---|---|
| 5.75 | 423.32 | 0.0768 | 15.38 | 51.60 |
| 8.23 | 74.27 | 0.2047 | 10.75 | 9.05 |
| 9.14 | 278.10 | 0.1023 | 9.67 | 33.90 |
| 9.69 | 57.21 | 0.1535 | 9.13 | 6.97 |
| 13.71 | 434.75 | 0.0768 | 6.46 | 52.99 |
| 15.28 | 89.43 | 0.1023 | 5.80 | 10.90 |
| 16.27 | 90.44 | 0.0768 | 5.45 | 11.02 |
| 16.65 | 268.17 | 0.0768 | 5.33 | 32.69 |
| 17.17 | 371.35 | 0.1279 | 5.16 | 45.26 |
| 18.05 | 241.81 | 0.0768 | 4.91 | 29.47 |
| 18.29 | 820.39 | 0.1023 | 4.85 | 100.00 |
| 19.46 | 95.74 | 0.1535 | 4.56 | 11.67 |
| 20.18 | 428.58 | 0.1535 | 4.40 | 52.24 |
| 20.60 | 212.87 | 0.1023 | 4.31 | 25.95 |
| 21.79 | 148.23 | 0.1023 | 4.08 | 18.07 |
| 22.92 | 634.67 | 0.1279 | 3.88 | 77.36 |
| 23.36 | 99.40 | 0.1535 | 3.81 | 12.12 |
| 23.96 | 561.62 | 0.1279 | 3.71 | 68.46 |
| 24.76 | 284.98 | 0.1535 | 3.60 | 34.74 |
| 25.49 | 72.78 | 0.1535 | 3.49 | 8.87 |
| 26.07 | 126.02 | 0.1535 | 3.42 | 15.36 |
| 26.63 | 91.36 | 0.1535 | 3.35 | 11.14 |
| 27.64 | 83.91 | 0.1535 | 3.23 | 10.23 |
| 28.04 | 253.71 | 0.1279 | 3.18 | 30.93 |
| 29.18 | 477.61 | 0.1023 | 3.06 | 58.22 |
| 29.82 | 87.63 | 0.1535 | 3.00 | 10.68 |
| 30.92 | 65.42 | 0.2047 | 2.89 | 7.97 |
| 31.67 | 52.63 | 0.1535 | 2.83 | 6.42 |
| 32.32 | 138.25 | 0.1023 | 2.77 | 16.85 |
| 32.49 | 196.14 | 0.0768 | 2.76 | 23.91 |

In some embodiments of the present disclosure, when the crystal form E is subjected to thermogravimetric analysis (TGA), the crystal form E heated to 80°C has a weight loss of 1.9%, and the crystal form E heated from 80°C to 150°C has a weight loss of 4.9%, with an error tolerance of±0.1%.

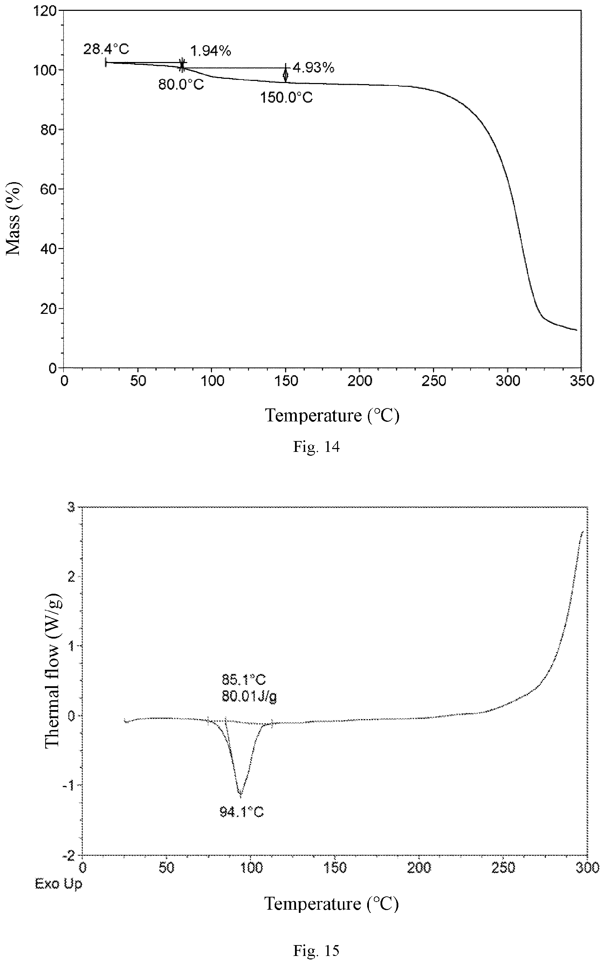

In some embodiments of the present disclosure, the crystal form E has a thermogravimetric analysis substantially as shown in Fig. 14.

In some embodiments of the present disclosure, the differential scanning calorimetry (DSC) pattern of the crystal form E has a wide endothermic peak at 94.1±3°C.

In some embodiments of the present disclosure, the crystal form E has a DSC differential scanning calorimetry curve substantially as shown in Fig. 15.

In some embodiments of the present disclosure, the crystal form E is a tetrahydrofuran solvate, and the content of the tetrahydrofuran is 2wt% to 14wt%, with an error tolerance of ±0.2%.

In some embodiments of the present disclosure, the crystal form E is a tetrahydrofuran solvate, and the content of the tetrahydrofuran is 2.1wt%, 2.3wt%, 2.5wt%, 2.8wt%, 3.1wt%, 3.3wt%, 3.5wt%, 3.8wt%, 4.1wt%, 4.3wt%, 4.5wt%, 4.8wt%, 5.1wt%, 5.3wt%, 5.5wt%, 5.8wt%, 6.1wt%, 6.3wt%, 6.5wt%, 6.8wt%, 7.1wt%, 7.3wt%, 7.4wt%, 7.5wt%, 7.8wt%, 8.1wt%, 8.3wt%, 8.5wt%, 8.8wt%, 9.1wt%, 9.3wt%, 9.5wt%, 9.8wt%, 10.1wt%, 10.3wt%, 10.5wt%, 10.7wt%, 10.9wt%, 11.1wt%, 11.3wt%, 11.5wt%, 11.8wt%, 12.1wt%, 12.3wt%, 12.5wt%, 12.8wt%, 13.1wt%, 13.3wt%, 13.5wt%, 13.6wt%, 13.8wt%, or 14.1wt%, with an error tolerance of±0.02%.

In one aspect of the present disclosure, the present disclosure provides a crystal form F of the compound represented by formula (I), the X-ray powder diffraction pattern thereof comprises characteristic diffraction peaks at the following angles of 2θ: 17.24±0.2°, 20.28±0.2°, 23.03±0.2°, 23.96±0.2°, 24.89±0.2° and 28.96±0.2°.

In some embodiments of the present disclosure, the X-ray powder diffraction pattern of the crystal form F comprises characteristic diffraction peaks at the following angles of 2θ: 5.78±0.2°, 14.31±0.2°, 17.24±0.2°, 20.28±0.2°, 22.06±0.2 °, 23.03±0.2°, 23.96±0.2°, 24.89±0.2°, 26.27±0.2° and 28.96±0.2°.

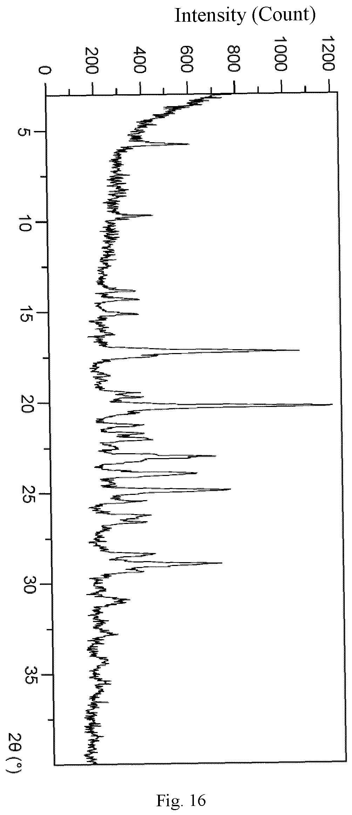

In some embodiments of the present disclosure, the X-ray powder diffraction pattern of the crystal form F has an X-ray powder diffraction pattern substantially as shown in Fig. 16.

In some embodiments of the present disclosure, the analytical data of the X-ray powder diffraction pattern of the crystal form F is as shown in Table 6.

**Table 6**

| 2θ (degrees) | Peak height (count) | Left half-peak width (2θ, degrees) | Interplanar spacing (Å) | Relative intensity (%) |
|---|---|---|---|---|
| 5.78 | 239.22 | 0.1023 | 15.30 | 24.50 |
| 9.72 | 155.38 | 0.1023 | 9.10 | 15.91 |
| 13.86 | 142.42 | 0.0768 | 6.39 | 14.59 |
| 14.31 | 153.72 | 0.1023 | 6.19 | 15.74 |
| 15.12 | 151.09 | 0.1279 | 5.86 | 15.47 |
| 16.21 | 49.24 | 0.1535 | 5.47 | 5.04 |
| 17.24 | 805.54 | 0.1279 | 5.14 | 82.50 |
| 17.48 | 231.23 | 0.1023 | 5.07 | 23.68 |
| 18.47 | 24.44 | 0.1535 | 4.80 | 2.50 |
| 19.47 | 167.16 | 0.0768 | 4.56 | 17.12 |
| 19.71 | 177.73 | 0.0768 | 4.50 | 18.20 |
| 20.28 | 976.41 | 0.1023 | 4.38 | 100.00 |
| 21.28 | 159.45 | 0.1535 | 4.18 | 16.33 |
| 21.73 | 196.57 | 0.0768 | 4.09 | 20.13 |
| 22.06 | 222.84 | 0.1791 | 4.03 | 22.82 |
| 22.49 | 83.39 | 0.1535 | 3.95 | 8.54 |
| 23.03 | 461.85 | 0.1023 | 3.86 | 47.30 |
| 23.96 | 419.97 | 0.2047 | 3.71 | 43.01 |
| 24.89 | 565.92 | 0.1535 | 3.58 | 57.96 |
| 25.48 | 196.78 | 0.1535 | 3.50 | 20.15 |
| 26.27 | 230.50 | 0.1023 | 3.39 | 23.61 |
| 26.62 | 205.17 | 0.1023 | 3.35 | 21.01 |
| 28.39 | 253.15 | 0.1535 | 3.14 | 25.93 |
| 28.96 | 534.42 | 0.1023 | 3.08 | 54.73 |
| 30.35 | 43.92 | 0.2047 | 2.94 | 4.50 |
| 30.90 | 130.26 | 0.1791 | 2.89 | 13.34 |
| 32.80 | 86.80 | 0.2047 | 2.73 | 8.89 |
| 34.32 | 46.84 | 0.4093 | 2.61 | 4.80 |
| 35.45 | 39.99 | 0.2047 | 2.53 | 4.10 |

In some embodiments of the present disclosure, when the crystal form F is subjected to thermogravimetric analysis (TGA), the crystal form F heated to 150°C has a weight loss of 11.5%, with an error tolerance of±0.1%.

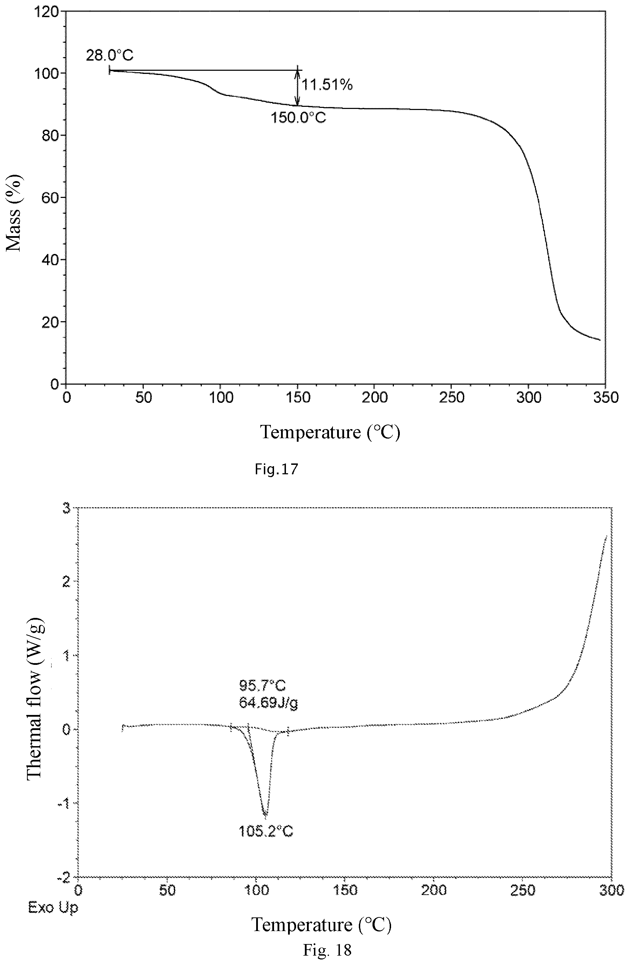

In some embodiments of the present disclosure, the crystal form F has a thermogravimetric analysis substantially as shown in Fig. 17.

In some embodiments of the present disclosure, the differential scanning calorimetry (DSC) pattern of the crystal form F has an endothermic peak at 105.2°C±3°C.

In some embodiments of the present disclosure, the crystal form F has a DSC differential scanning calorimetry curve substantially as shown in Fig. 18.

In some embodiments of the present disclosure, the crystal form F is a chloroform solvate, and the content of the chloroform is 5wt% to 21wt%, with an error tolerance of ±0.2%.

In some embodiments of the present disclosure, the crystal form F is a chloroform solvate, and the content of the chloroform is 11wt% to 21wt%, with an error tolerance of ±0.2%.

In some embodiments of the present disclosure, the crystal form F is a chloroform solvate, and the content of the chloroform is 5.1wt%, 5.3wt%, 5.5wt%, 5.8wt%, 6.1wt%, 6.3wt%, 6.5wt%, 6.8wt%, 7.1wt%, 7.3wt%, 7.4wt%, 7.5wt%, 7.8wt%, 8.1wt%, 8.3wt%, 8.5wt%, 8.8wt%, 9.1wt%, 9.3wt%, 9.5wt%, 9.8wt%, 10.1wt%, 10.3wt%, 10.5wt%, 10.7wt%, 10.9wt%, 11.1wt%, 11.3wt%, 11.5wt%, 11.8wt%, 12.1wt%, 12.3wt%, 12.5wt%, 12.8wt%, 13.1wt%, 13.3wt%, 13.5wt%, 13.6wt%, 13.8wt%, 14.1wt%, 14.3wt%, 14.5wt%, 14.8wt%, 15.1wt%, 15.3wt%, 15.5wt%, 15.8wt%, 16.1wt%, 16.3wt%, 16.5wt%, 16.8wt%, 17.1wt%, 17.3wt%, 17.5wt%, 17.8wt%, 18.1wt%, 18.3wt%, 18.5wt%, 18.8wt%, 19.1wt%, 19.3wt%, 19.5wt%, 19.8wt%, 20.1wt%, 20.3wt%, 20.5wt%, 20.6wt%, 20.7wt%, 20.8wt% or21.1wt%, with an error tolerance of ±0.02%.

In one aspect of the present disclosure, the present disclosure provides a crystal form G of the compound represented by formula (I), the X-ray powder diffraction pattern thereof comprises characteristic diffraction peaks at the following angles of 2θ: 15.53±0.2°, 17.08±0.2°, 21.41±0.2°, 23.23±0.2°, 26.00±0.2° and 28.49±0.2°

In some embodiments of the present disclosure, the X-ray powder diffraction pattern of the crystal form G comprises characteristic diffraction peaks at the following angles of 2θ: 10.54±0.2°, 13.02±0.2°, 15.53±0.2°, 17.08±0.2°, 21.41±0.2 °, 23.23±0.2°, 25.10±0.2°, 26.00±0.2°, 27.17±0.2° and 28.49±0.2°.

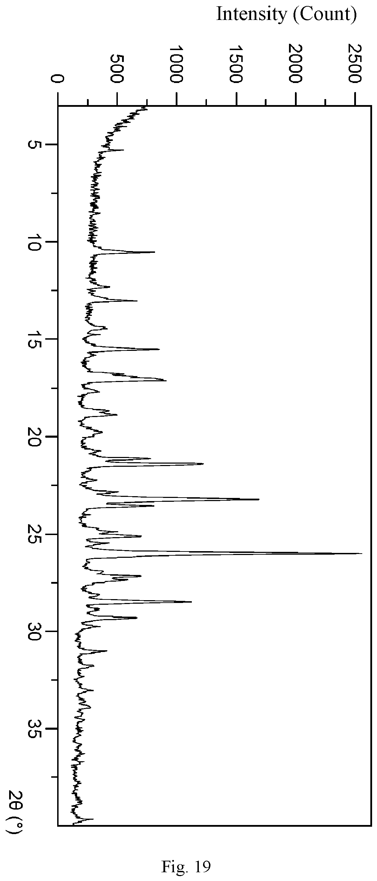

In some embodiments of the present disclosure, the X-ray powder diffraction pattern of the crystal form G has an X-ray powder diffraction pattern substantially as shown in Fig. 19.

In some embodiments of the present disclosure, the analytical data of the X-ray powder diffraction pattern of the crystal form G is as shown in Table 7.

**Table 7**

| 2θ (degrees) | Peak height (count) | Left half-peak width (2θ, degrees) | Interplanar spacing (Å) | Relative intensity (%) |
|---|---|---|---|---|
| 5.26 | 166.80 | 0.1535 | 16.79 | 7.21 |
| 10.54 | 521.78 | 0.1023 | 8.39 | 22.56 |
| 12.28 | 149.34 | 0.1535 | 7.21 | 6.46 |
| 13.02 | 404.54 | 0.1023 | 6.80 | 17.49 |
| 14.42 | 160.92 | 0.1535 | 6.14 | 6.96 |
| 15.53 | 625.10 | 0.1023 | 5.71 | 27.02 |
| 17.08 | 661.47 | 0.1279 | 5.19 | 28.60 |
| 17.68 | 132.53 | 0.2047 | 5.02 | 5.73 |
| 18.89 | 303.22 | 0.1023 | 4.70 | 13.11 |
| 19.77 | 156.95 | 0.3070 | 4.49 | 6.78 |
| 21.13 | 585.57 | 0.0768 | 4.20 | 25.31 |
| 21.41 | 1033.95 | 0.1279 | 4.15 | 44.70 |
| 22.24 | 130.35 | 0.1535 | 4.00 | 5.64 |
| 22.85 | 311.38 | 0.1023 | 3.89 | 13.46 |
| 23.23 | 1481.52 | 0.1535 | 3.83 | 64.05 |
| 23.57 | 613.05 | 0.1023 | 3.78 | 26.50 |
| 25.10 | 518.34 | 0.1023 | 3.55 | 22.41 |
| 25.47 | 235.60 | 0.1023 | 3.50 | 10.18 |
| 26.00 | 2313.18 | 0.1023 | 3.43 | 100.00 |
| 27.17 | 515.97 | 0.1023 | 3.28 | 22.31 |
| 28.49 | 904.11 | 0.1279 | 3.13 | 39.09 |
| 28.89 | 163.06 | 0.1535 | 3.09 | 7.05 |
| 29.32 | 495.64 | 0.1023 | 3.05 | 21.43 |
| 29.75 | 139.77 | 0.1535 | 3.00 | 6.04 |
| 31.02 | 245.24 | 0.1279 | 2.88 | 10.60 |
| 31.79 | 132.59 | 0.1535 | 2.82 | 5.73 |
| 33.05 | 97.60 | 0.1535 | 2.71 | 4.22 |
| 33.88 | 113.47 | 0.1535 | 2.65 | 4.91 |

In some embodiments of the present disclosure, when the crystal form G is subjected to thermogravimetric analysis (TGA), the crystal form G heated to 160°C has a weight loss of 2.3%, with an error tolerance of±0.1%.

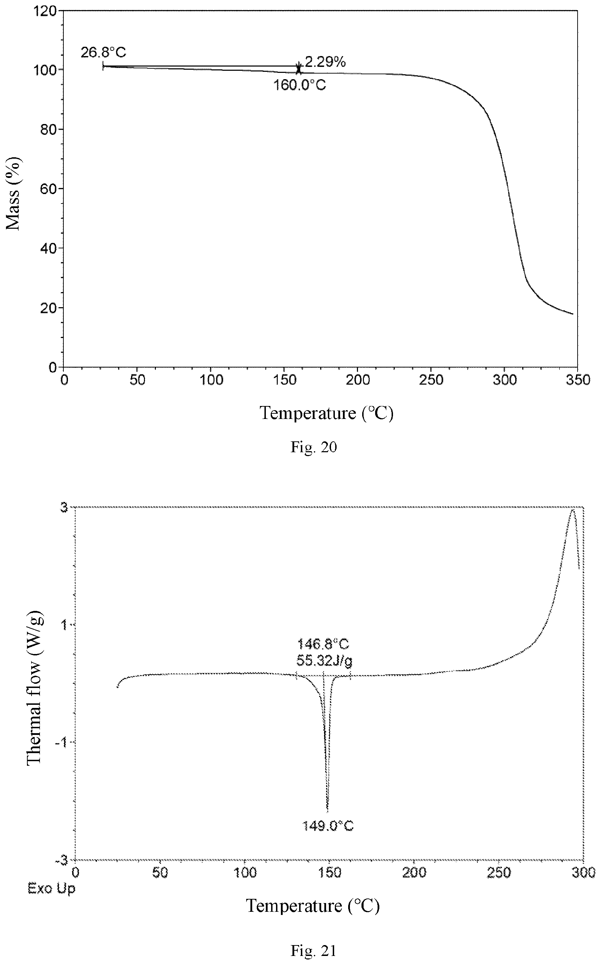

In some embodiments of the present disclosure, the crystal form G has a thermogravimetric analysis substantially as shown in Fig. 20.

In some embodiments of the present disclosure, the differential scanning calorimetry (DSC) pattern of the crystal form G has an endothermic peak at 149.0°C±3°C.

In some embodiments of the present disclosure, the crystal form G has a DSC differential scanning calorimetry curve substantially as shown in Fig. 21.

In some embodiments of the present disclosure, the crystal form G is an anhydrous crystal form.

In another aspect of the present disclosure, the present disclosure also discloses a pharmaceutical composition. In some embodiments of the present disclosure, the pharmaceutical composition comprises the crystal form A to G.

In some embodiments of the present disclosure, the pharmaceutical composition further comprises a pharmaceutically acceptable carrier, excipient, diluent, adjuvant, vehicle or their combination.

In another aspect of the present disclosure, the present disclosure also discloses a use of the crystal forms A to G or the pharmaceutical composition in the manufacture of a medicament for inhibiting a voltage-gated sodium channel of individual.

In some embodiments of the present disclosure, the voltage-gated sodium channel is NaVl.8.

In another aspect of the present disclosure, the present disclosure also discloses a use of the crystal forms A to G in the manufacture of a medicament for treating and/or preventing pain, cough or alleviating their severity of individual.

In some embodiments of the present disclosure, the pain is selected from chronic pain, bowel pain, neuropathic pain, musculoskeletal pain, acute pain, inflammatory pain, cancer pain, primary pain, postoperative pain, visceral pain, multiple sclerosis, Charcot-Marie-Tooth syndrome, incontinence and arrhythmia.

In some embodiments of the present disclosure, the bowel pain is selected from inflammatory bowel disease pain, Crohn's disease pain and interstitial cystitis pain.

In some embodiments of the present disclosure, the neuropathic pain is selected from postherpetic neuralgia, diabetic neuralgia, painful HIV-related sensory neuropathy, trigeminal neuralgia, mouth burn syndrome, postoperative pain after amputation, phantom pain, painful neuroma, traumatic neuroma, Morto neuroma, nerve crush injury, spinal stenosis, carpal tunnel syndrome, radiculalgia, sciatica, nerve avulsion, brachial plexus avulsion, complex regional pain syndrome, neuralgia caused by drug therapy, neuralgia caused by cancer chemotherapy, neuralgia caused by antiretroviral therapy, pain after spinal cord injury, primary small fiber neuropathy, primary sensory neuropathy, and trigeminal autonomic headache.

In some embodiments of the present disclosure, the musculoskeletal pain is selected from osteoarthritis pain, backache, cold pain, burn pain and toothache.

In some embodiments of the present disclosure, the inflammatory pain is selected from rheumatoid arthritis pain and vulvodynia.

In some embodiments of the present disclosure, the primary pain is selected from fibromyalgia.

In another aspect of the present disclosure, the present disclosure also provides a method for treating or alleviating the pain in a subject.

In some embodiments of the present disclosure, the method comprises administering a therapeutically effective amount of the crystal forms A to G or the pharmaceutical composition. In some embodiments of the present disclosure, the pain in the subject is as defined herein.

In another aspect of the present disclosure, the present disclosure also provides a method for inhibiting the voltage-gated sodium channel in a subject.

In some embodiments of the present disclosure, the method comprises administering a therapeutically effective amount of the crystal forms A to G or the pharmaceutical composition. In some embodiments of the present disclosure, the voltage-gated sodium channel is NaV1.8.

### Definition and description

Unless otherwise specified, all of the technical and scientific terms used in the present disclosure have the same meaning as commonly understood by those skilled in the art to which the present disclosure belongs. All patents and publications referred to herein are incorporated in their entirety by reference. Although any methods and materials similar or identical to those described herein can be used in the practice or testing of the present disclosure, the preferred methods, devices and materials are described herein.

"Crystal" or "crystal form" refers to a solid with a highly regular chemical structure, including, but not limited to, single-component or multi-component crystal, and/or polymorph, solvate, hydrate, clathrate, co-crystals, salt, solvate of salt, hydrate of salt of the compound. The crystal form of the substance can be obtained by many methods known in the art. These methods include, but are not limited to, melt crystallization, melt cooling, solvent crystallization, crystallization in a confined space, for example, in nanopores or capillaries, crystallization on the surface or template, for example, on polymers, crystallization in the presence of additives such as co-crystallized antimolecule, desolvation, dehydration, rapid evaporation, rapid cooling, slow cooling, vapor diffusion, sublimation, reaction crystallization, addition of anti-solvent, grinding and solvent drop grinding .

"Amorphism" or "amorphous form" refers to a substance formed when its particles (molecules, atoms, ions) are arranged in three-dimensional space without periodicity, which is characterized by a diffuse X-ray powder diffraction pattern without sharp peaks. Amorphous is a special physical form of solid substance, and its local ordered structural features suggest that it is inextricably linked with crystal substances. The amorphous form of the substance can be obtained by many methods known in the art. These methods include, but are not limited to, shock-cooling method, anti solvent flocculation method, ball milling method, spray drying method, freeze drying method, wet granulation method and solid dispersion technology, etc.

"Solvent" refers to a substance (typically a liquid) that can completely or partially dissolve another substance (typically a solid). Solvents used in the practice of the present disclosure include, but are not limited to, water, acetic acid, acetone, acetonitrile, benzene, chloroform, carbon tetrachloride, dichloromethane, dimethyl sulfoxide, 1,4-dioxane, ethanol, ethyl acetate, butanol, tert-butanol, *N,N-*dimethylacetamide, *N*,*N*-dimethylformamide, formamide, formic acid, heptane, hexane, isopropanol, methanol, methyl ethyl ketone, 1-methyl-2-pyrrolidone, mesitylene, nitromethane, polyethylene glycol, propanol, 2-propanone, pyridine, tetrahydrofuran, toluene, xylene, mixtures thereof, etc.

"Antisolvent" refers to a fluid that facilitates precipitation of a product (or product precursor) from a solvent. The anti solvent may comprise a cold gas, or a fluid that promotes precipitation through a chemical reaction, or a fluid that reduces the solubility of the product in the solvent; it can be the same liquid as the solvent but at a different temperature, or it can be a different liquid than the solvent.

"Solvate" means that the crystal has a solvent on the surface, or in the lattice, or both on the surface and in the lattice, wherein the solvent can be water, acetic acid, acetone, acetonitrile, benzene, chloroform, carbon tetrachloride, dichloromethane, dimethyl sulfoxide, 1,4-dioxane, ethanol, ethyl acetate, butanol, *tert*-butanol, *N*,*N*-dimethylacetamide, *N*,*N*-dimethylformamide, formamide, formic acid, heptane, hexane, isopropanol, methanol, methyl ethyl ketone, methyl-pyrrolidone, mesitylene, nitromethane, polyethylene glycol, propanol, 2-propanone, pyridine, tetrahydrofuran, toluene, xylene and the mixtures thereof, etc. A specific example of a solvate is hydrate, wherein the solvent on the surface, or in the lattice, or on the surface and in the lattice, is water. On the surface of the substance, or in the lattice, or on the surface and in the lattice, the hydrate may or may not have a solvent other than water.

The crystal form or amorphous form can be identified by various techniques, such as X-ray powder diffraction (XRPD), infrared absorption spectroscopy (IR), melting point method, differential scanning calorimetry (DSC), thermogravimetric analysis (TGA), nuclear magnetic resonance, Raman spectroscopy, X-ray single crystal diffraction, dissolution calorimetry, scanning electron microscopy (SEM), quantitative analysis, solubility and dissolution rate, etc.

X-ray powder diffraction (XRPD) can detect the change of crystal form, crystallinity, crystal structure and other information, and is a common method for identifying crystal form. The peak positions of the XRPD patterns depend primarily on the structure of the crystal form and are relatively insensitive to experimental details, while their relative peak heights depend on many factors related to sample preparation and instrument geometry. Therefore, in some embodiments, the crystal form of the present disclosure is characterized by an XRPD pattern with certain peak positions, which is substantially as shown in the XRPD pattern provided in the drawings of the present disclosure. At the same time, the measurement of 2θ of XRPD pattern may have experimental errors, and the measurement of 2θ of XRPD spectrum may be slightly different between different instruments and different samples, so the value of 2θ cannot be regarded as absolute. According to the condition of the instrument used in the experiment of the present disclosure, the diffraction peak has an error tolerance of±0.2°.

Differential scanning calorimetry (DSC) is a program-controlled technique that measures the energy difference between a sample and an inert reference (commonly α-Al₂O₃) as a function of temperature by continuously heating or cooling. The melting peak height of the DSC curve depends on many factors related to sample preparation and instrument geometry, while the peak position is relatively insensitive to experimental details. Therefore, in some embodiments, the crystal form of the present disclosure is characterized by an DSC pattern with characteristic peak positions, which is substantially as shown in the DSC pattern provided in the drawings of the present disclosure. At the same time, the DSC pattern may have experimental errors, and the peak position and the peak value of DSC pattern may be slightly different between different instruments and different samples, so the peak position and the peak value of DSC endothermic peak cannot be regarded as absolute. According to the condition of the instrument used in the experiment of the present disclosure, the melting peak has an error tolerance of±3°C.

Glass transition refers to the transition of an amorphous substance between a highly elastic state and a glass state, which is the inherent property of the substance; and its corresponding transition temperature is the glass transition temperature (Tg), which is an important physical property of amorphous substances. The glass transition is a phenomenon related to molecular motion, and thus the glass transition temperature (Tg) depends mainly on the structure of the substance and is relatively insensitive to experimental details, etc. In some embodiments, the amorphous glass transition temperature (Tg) of the present disclosure is determined by differential scanning calorimetry (DSC) and wherein, the amorphous glass transition temperature has a glass transition temperature of 107.44°C. According to the condition of the instrument used in the experiment of the present disclosure, the glass transition temperature has an error tolerance of±3°C.

Differential scanning calorimetry (DSC) can also be used to detect and analyze whether the crystal form has the phenomenon of crystalline transformation or mixed crystal.

Solids with the same chemical composition, under different thermodynamic conditions, often form homomers with different crystal structures, or variants, which is called polymorphism or homogeneous polyphase phenomenon. When temperature and pressure conditions change, the variants will transform into each other, which is called crystal transformation. Due to the crystal transformation, the mechanical, electrical and magnetic properties of the crystal will change greatly. When the temperature of the crystal transformation is in the measurable range, the transformation process can be observed on a differential scanning calorimetry (DSC) pattern, wherein, the DSC pattern has an exothermic peak reflecting this transformation process, and there are two or more endothermic peaks at the same time, which are the characteristic endothermic peaks of different crystal forms before and after transformation. The crystal form or amorphous form of the compound of the present disclosure can undergo crystal form transformation under appropriate conditions.

Thermogravimetric analysis (TGA) is a technique for determining the mass of a substance with temperature under program control, which is suitable for checking the loss of solvent in crystal or the process of sublimation and decomposition of samples, and can infer the presence of crystal water or crystalline solvents in crystals. The mass change shown by the TGA curve depends on many factors such as sample preparation and instrument; the mass change detected by TGA varies slightly between different instruments and between different samples. In some embodiments, the calcium salt crystal form A of the present disclosure loses about 5.1% weight at about 150°C. According to the condition of the instrument used in the experiment of the present disclosure, the mass change has an error tolerance of±0.3%.

In the context of the present disclosure, the 2θ values in the X-ray powder diffraction pattern are all in degrees (°).

It should be noted that "wt%" refers to the mass ratio (g/g); for example, in hydrate, the moisture content of crystal form A is 3.0wt%, which means that the ratio of the mass of water in crystal form A to the mass of crystal form A (g/g) is 3.0; for example, in solvents, the content of 1,4-dioxane in crystal form C is 3.1wt%, which means that the mass of 1,4-dioxane in crystal form C and the mass of crystal form C (g/g) is 3.1.

The term "substantially as shown" means at least 50%, or at least 60%, or at least 70%, or at least 80%, or at least 90%, or at least 95%, or at least 99% of the peaks in the X-ray powder diffraction pattern or DSC pattern or TGA result, are shown in the diagram.

When referring to a pattern or/and data appearing in the pattern, "peak" refers to a feature that can be recognized by those skilled in the art and is not attributed to background noise.

"Substantially pure" means that a crystal form is substantially free of one or more other crystal forms; for example, the purity of crystal form is at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or the crystal form contains other crystal forms, the percentage of the other crystal forms in the total volume or the total weight of the crystal form is less than 20%, or less than 10%, or less than 5%, or less than 3%, or less than 1%, or less than 0.5%, or less than 0.1%, or less than 0.01%.

"Substantially free" means that the percentage of one or more other crystal forms in the total volume or the total weight of the crystal form is less than 20%, or less than 10%, or less than 5%, or less than 4%, or less than 3%, or less than 2%, or less than 1%, or less than 0.5%, or less than 0.1%, or less than 0.01%.

"Relative intensity" refers to the ratio of the intensity of the other peaks to the intensity of the first strong peak when the intensity of the first strong peak among all diffraction peaks in the X-ray powder diffraction pattern (XRPD) is 100%.

In the context of the present disclosure, when the word "about" or "approximately" is used or not, it means within 10% of a given value or range, appropriately within 5%, especially within 1%. Or, for those skilled in the art, the term "about" or "approximately" means that it is within the acceptable standard error range of the average value. Whenever a number with a value of N is disclosed, any number with N+/-1%, N+/-2%, N+/-3%, N+/-5%, N+/-7%, N+/-8% or N+/-Numbers within 10% of the value are explicitly disclosed, wherein "+/-" means plus or minus.

The term "comprises" is an open definition; that is, it includes the contents specified in the present disclosure but does not exclude other aspects.

### BRIEF DESCRIPTION OF THE DRAWINGS

Fig. 1 is the X-ray powder diffraction (XRPD) pattern of the crystal form A;
Fig. 2 is the thermogravimetric analysis (TGA) pattern of the crystal form A;
Fig. 3 is the differential scanning calorimetry (DSC) pattern of the crystal form A;
Fig. 4 is the X-ray powder diffraction (XRPD) pattern of the crystal form B;
Fig. 5 is the thermogravimetric analysis (TGA) pattern of the crystal form B;
Fig. 6 is the differential scanning calorimetry (DSC) pattern of the crystal form B;
Fig. 7 is the X-ray powder diffraction (XRPD) pattern of the crystal form C;
Fig. 8 is the thermogravimetric analysis (TGA) pattern of the crystal form C;
Fig. 9 is the differential scanning calorimetry (DSC) pattern of the crystal form C;
Fig. 10 is the X-ray powder diffraction (XRPD) pattern of the crystal form D;
Fig. 11 is the thermogravimetric analysis (TGA) pattern of the crystal form D;
Fig. 12 is the differential scanning calorimetry (DSC) pattern of the crystal form D;
Fig. 13 is the X-ray powder diffraction (XRPD) pattern of the crystal form E;
Fig. 14 is the thermogravimetric analysis (TGA) pattern of the crystal form E;
Fig. 15 is the differential scanning calorimetry (DSC) pattern of the crystal form E;
Fig. 16 is the X-ray powder diffraction (XRPD) pattern of the crystal form F;
Fig. 17 is the thermogravimetric analysis (TGA) pattern of the crystal form F;
Fig. 18 is the differential scanning calorimetry (DSC) pattern of the crystal form F;
Fig. 19 is the X-ray powder diffraction (XRPD) pattern of the crystal form G;
Fig. 20 is the thermogravimetric analysis (TGA) pattern of the crystal form G;
Fig. 21 is the differential scanning calorimetry (DSC) pattern of the crystal form G;
Fig. 22 is the ¹H NMR pattern of the crystal form B;
Fig. 23 is the ¹H NMR pattern of the crystal form C;
Fig. 24 is the ¹H NMR pattern of the crystal form E;
Fig. 25 is the ¹H NMR pattern of the crystal form F;
Fig. 26 is the ¹H NMR pattern of the crystal form G;
Fig. 27 is the XRPD stack pattern (VIII) of the solids after suspension competition;

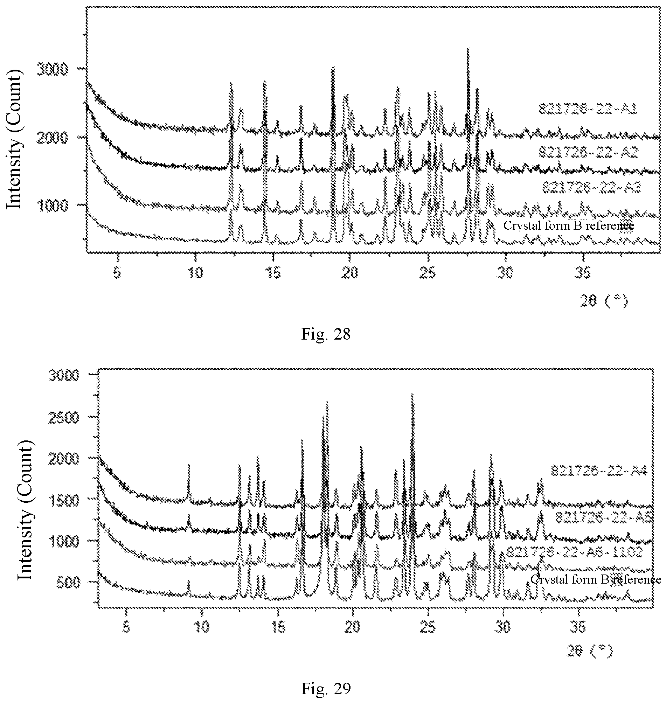

Fig. 28 is the XRPD stack pattern (II/III) of the solids after suspension competition;
Fig. 29 is the XRPD stack pattern (III/III) of the solids after suspension competition.

### DETAILED DESCRIPTION OF THE PREFERRED EMBODIMENT

The present disclosure is described in detail by the embodiments below, but it does not mean that there are any adverse restrictions on the present disclosure. The present disclosure has been described in detail herein, wherein specific embodiments thereof are also disclosed, and it will be apparent to those skilled in the art that various variations and improvements can be made to specific embodiments of the present disclosure without departing from the spirit and scope of the present disclosure.

The XRPD results of the present disclosure were collected on PANalytical Empyrean and X'Pert3 X-ray powder diffraction analyzer, and the scanning parameters are shown in the following Table 8.

**Table 8**

| Instrument model | X' Pert³ (reflection) | Empyrean (reflection/variable temperature) |
|---|---|---|
| X-ray | Cu, kα; Kα1 (Å): 1.540598, Kα2 (Å): 1.544426 intensity ratio of Kα2/Kα1: 0.50 | Cu, kα; Kα1 (Å): 1.540598, Kα2 (Å): 1.544426 intensity ratio of Kα2/Kα1: 0.50 |
| X-ray tube setting | 45 kV, 40 mA | 45 kV, 40 mA |
| Divergent slit | 1/8° | Automatic |
| Scanning type | Continuous | Continuous |
| Scanning range (°2TH) | 3° to 40° | 3° to 40° |
| Scanning step time (°2TH) | 0.0263 | 0.0167 |
| Scanning time per step (s) | 46.665 | 17.780/33.020 |
| Scanning time (s) | 5 min 03 s | 5 min 32 s/10 min 13 s |

The TGA and DSC patterns of the present disclosure are collected on the TA Q5000/Discovery 5500 thermogravimetric analyzer and TA Discovery 2500 differential scanning calorimeter, respectively, and the test parameters are listed in Table 9.

**Table 9**

| | | |
|---|---|---|
| Parameter | TGA | DSC |
| Method | Linear heating | Linear heating |
| Sample tray | Aluminum tray, opening | Aluminum tray, glanding |
| Temperature range | RT-350°C | RT-target temperature |
| Heating rate | 10°C/min | 10°C/min |
| Protective gas | Nitrogen | Nitrogen |

The dynamic moisture adsorption (DVS) curve of the present disclosure is collected on DVS Intrinsic of SMS (Surface Measurement Systems). The relative humidity at 25°C is corrected with the deliquescence point of LiCl, Mg(NO₃)₂ and KCl. The DVS test parameters are listed in Table 10.

**Table 10**

| | |
|---|---|
| Parameter | DVS |
| Temperature | 25°C |
| Sample volume | 20 to 40 mg |
| Protective gas and flow rate | N₂, 200 mL/min |
| dm/dt | 0.002%/min |
| Minimum dm/dt equilibration time | 10mm |
| Maximum equilibration time | 180min |
| RH range | 0%RH to 95%RH |
| RH gradient | 0% to 0%RH to 95%RH |
| | 10%RH (0%RH to 90%RH & 90%RH to 0%RH) |
| | 5%RH (90%RH to 95%RH & 95%RH to 90%RH) |

The hydrogen spectrum liquid nuclear magnetic spectrum of the present disclosure is collected on a Bruker 400M nuclear magnetic resonance spectrometer, and DMSO-*d*₆ is used as a solvent.

The stability test in the high-performance liquid chromatography (HPLC) test used in the present disclosure is tested by an Agilent 1260 high-performance liquid chromatograph, and the analytical conditions are shown in Table 11.

**Table 11**

| | | |
|---|---|---|
| HPLC | Agilent 1260 (DAD detector) | |
| Chromatographic column | Waters Xbridge C18, 150x4.6 mm, 5 µm | |
| Mobile phase | A 0.1% TFA in H₂O | |
| | B: 0.1% TFA in ACN | |
| Running gradient | Purity | |
| | Time (min) | %B |
| | 0.0 | 10 |
| | 5.0 | 10 |
| | 15.0 | 90 |
| | 20.0 | 90 |
| | 20.1 | 10 |
| | 25.0 | 10 |
| Running time | 25min | |
| Post-running time | 0.0min | |
| Mobile phase flow rate | 1.0 mL/min | |
| Injection volume | 5 µL | |
| Detection wavelength | UV at 254 nm | |
| Column temperature | 30°C | |
| Injector temperature | RT | |
| Diluent | ACN:H₂O (1:1, v:v) | |

The abbreviations or Chinese meanings of the solvent used in the present disclosure are shown in the following Table 12:

**Table 12**

| English | Chinese | English | Chinese |
|---|---|---|---|
| MeOH | Methanol | MTBE | Methyl tert-butyl ether |
| EtOH | Ethanol | THF | Tetrahydrofuran |
| IPA | Isopropanol | 2-MeTHF | 2-Methyltetrahydrofuran |
| CHCl₃ | Chloroform | ACN | Acetonitrile |
| MIBK | Methyl isobutyl ketone | n-Heptane | n-Heptane |
| EtOAc | Ethyl acetate | Toluene | Toluene |
| IPAc | Isopropyl acetate | H₂O | Water |
| DMSO | dimethyl sulfoxide | DCM | Dichloromethane |
| Anisole | Anisole | 1,4-Dioxane | 1,4-Dioxane |
| MEK | Methyl ethyl ketone | DMF | Dimethylformamide |
| Cumene | Cumene | n-Hexane | n-Hexane |

The embodiments of the present disclosure disclose the crystal forms of the compound of formula (I) and the preparation method thereof. Those skilled in the art can learn from the content of the present disclosure and appropriately improve the process parameters to achieve. It should be particularly pointed out that all similar substitutions and modifications will be apparent to those skilled in the art, and they are deemed to be included in the present disclosure. The methods of the present disclosure have been described through the preferred embodiments, and it is obvious that relevant persons are able to make modifications or appropriate changes to the methods described herein, or combine them, without departing from the content, spirit and scope of the present disclosure, so as to implement and apply the technology of the present disclosure.

In order to further understand the present disclosure, the present disclosure is described in detail below with reference to the embodiments.

### Embodiment 1: preparation of the compound of formula (I)

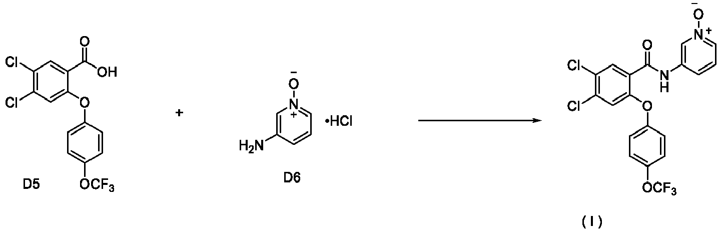

According to the method in WO 2019014352, the intermediate D5 was synthesized. 200 mL of dichloromethane was added into a reaction flask, and 40 g of D5, 53.8 g of HATU and 19.1 g of D6 were added into the reaction flask under the condition of stirring, and the reaction solution was stirred continuously. 42.2g of DIPEA was slowly added into the reaction flask, and the internal temperature of the reaction flask was not higher than 35°C during dropwise addition. After the reaction was completed, the internal temperature of the reaction flask was kept at 30 to 35°C and the reaction solution was stirred continuously for 16 hours. After the reaction was completed, the reaction system was cooled to 25 to 30° C, 100 mL of dichloromethane was added, and 240 mL of prepared 5% potassium carbonate aqueous solution was added to the system, the mixture was stirred for 0.5 hours, and then allowed to stand for liquid separation. The organic phase was washed with 5% citric acid aqueous solution, 7% sodium bicarbonate aqueous solution and purified water, in turn, and then concentrated under reduced pressure to obtain the compound of formula (I).

LCMS: m/z 459.0 (M+H) ⁺; ¹H NMR (400 MHz, DMSO-*d*₆) *δ* 10.83 (s, 1H), 8.61 (s, 1H), 8.03 (s, 1H), 8.00 - 7.98 (m, 1H), 7.47 - 7.44 (m, 2H), 7.40 - 7.34 (m, 3H), 7.21- 7.16 (m, 2H).

### Embodiment 2: preparation and identification of the crystal form A

20.2 mg of the compound of formula (I) was slurried in 0.3 mL of MTBE for 10 days at room temperature to obtain the crystal form A. The crystal form A of the XRPD results is shown in Fig. 1, the crystal form A of the TGA results is shown in Fig. 2, and the crystal form A of the DSC results is shown in Fig. 3. The TGA results showed that the sample heated to 150°C had a weight loss of 3.9% (the theoretical content of crystal water is about 3.8%). The DSC results showed that the sample has a wide endothermic peak at 86.3°C (initial temperature).

### Embodiment 3: preparation and identification of the crystal form B

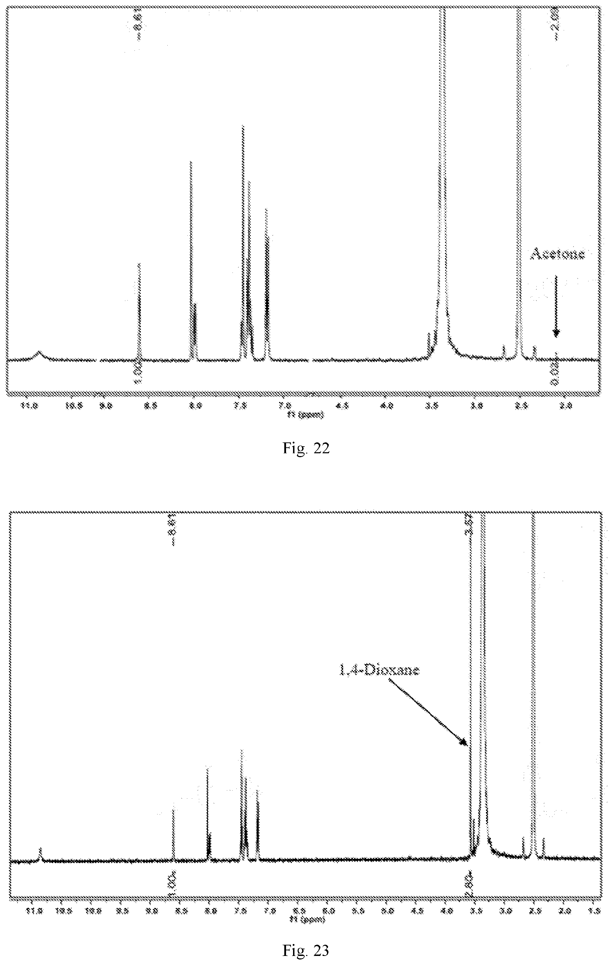

19.9 mg of the compound of formula (I) was slurried in 0.3 mL of acetone for 10 days at room temperature to obtain the crystal form B. The XRPD results are shown in Fig. 4, the TGA results are shown in Fig. 5, and the DSC results are shown in Fig. 6. The TGA results showed that the sample heated to 150°C had a weight loss of 1.2%. The DSC results showed that the sample has an endothermic peak at 146.6°C (initial temperature). ¹H NMR results (Fig. 22) showed that only a very small amount (0.04wt%) of acetone residue was observed in this sample.

### Embodiment 4: preparation and identification of the crystal form C

20.0 mg of the compound of formula (I) was slurried in 0.5 mL of 1,4-dioxane/toluene (1:4, v/v) for 10 days at room temperature to obtain the crystal form C. The XRPD results are shown in Fig. 7, the TGA results are shown in Fig. 8, and the DSC results are shown in Fig. 9. The TGA results showed that the sample heated to 80°C had a weight loss of 1.2%, and the sample heated to 150°C had a step weight loss of 7.0%. The DSC results showed that overlapping endothermic peaks were observed in the sample at 106.6°C and 111.3°C (peak temperature). ¹H NMR results showed (Fig.23) that 6.3wt% of 1,4-dioxane was detected.

### Embodiment 5: preparation and identification of the crystal form D

15.0 mg of the compound of formula (I) was gas-solid diffused in 4 mL of MEK for one week to obtain the crystal form B. The XRPD results are shown in Fig. 10, the TGA results are shown in Fig. 11, and the DSC results are shown in Fig. 12. The TGA results showed that the sample heated to 80°C had a weight loss of 0.9%, and the sample heated to 150°C had a weight loss of 7.0%. The DSC results showed that the sample had two endothermic peaks at 97.8°C and 149.2°C (peak temperature).

### Embodiment 6: preparation and identification of the crystal form E

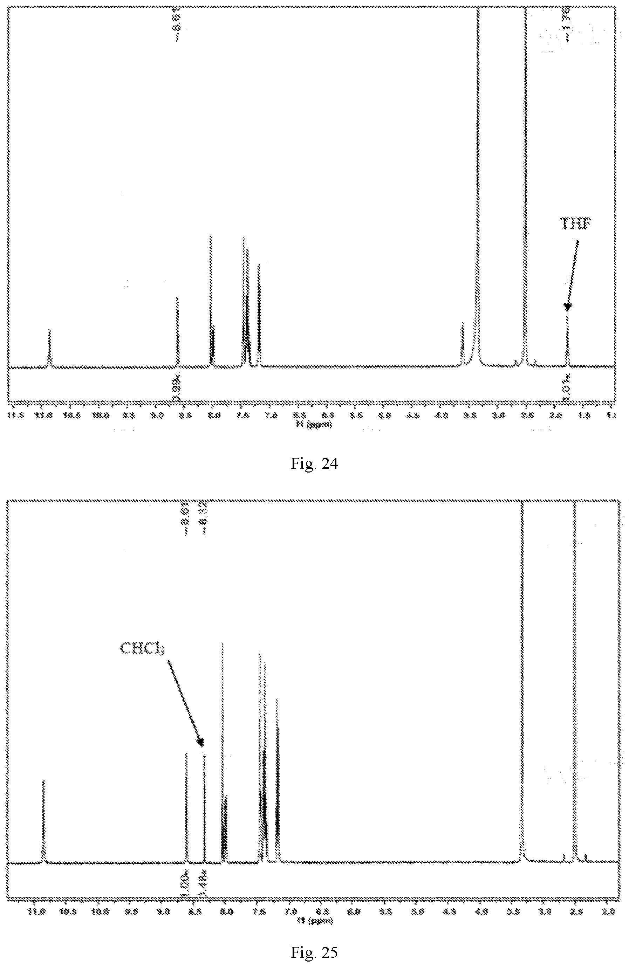

15.0 mg of the compound of formula (I) was gas-solid diffused in 4 mL of THF for one week at room temperature to obtain the crystal form E. The XRPD results are shown in Fig. 13, the TGA results are shown in Fig. 14, and the DSC results are shown in Fig. 15. The TGA results showed that the sample heated to 80°C had a weight loss of 1.9%, and the sample heated from 80°C to 150°C had a weight loss of 4.9%. The DSC results showed that the sample has a wide endothermic peak at 85.1°C (initial temperature). ¹H NMR results showed (Fig. 24) that 3.8wt% of THF was detected.

### Embodiment 7: preparation and identification of the crystal form F

15.1 mg of the compound of formula (I) was gas-solid diffused in 4 mL of CHCl₃ for one week at room temperature to obtain the crystal form F. The XRPD results are shown in Fig. 16, the TGA results are shown in Fig. 17, and the DSC results are shown in Fig. 18. The TGA results showed that the sample heated to 150°C had a weight loss of 11.5%. The DSC results showed that the sample has an endothermic peak at 95.7°C (initial temperature). ¹H NMR results showed (Fig. 25) that 11.1wt% of CHCl₃ was detected (consistent with the TGA weight loss).

### Embodiment 8: preparation and identification of the crystal form G

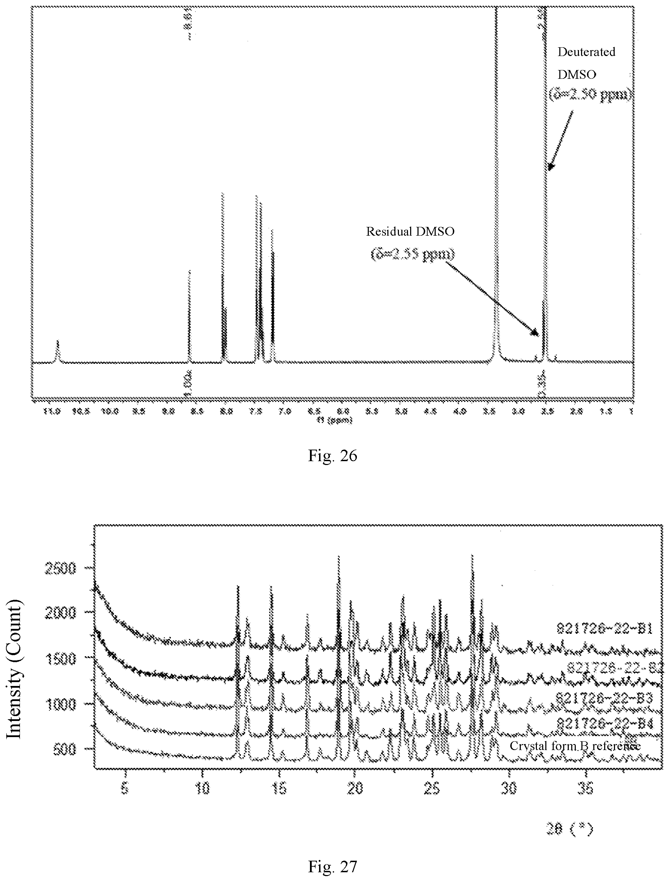

About 20 mg of the compound of formula (I) was gas-solid diffused in DMSO for about four days at room temperature, and the mixture was heated to 100 °C under the protection of nitrogen and then cooled to room temperature to obtain the crystal form G. The XRPD results are shown in Fig. 19, the TGA results are shown in Fig. 20, and the DSC results are shown in Fig. 21. The TGA results showed that the sample of the crystal form G heated to 160°C had a weight loss of 2.3%. The DSC results showed that the sample has an endothermic peak at 146.8°C (initial temperature). ¹H NMR results showed (Fig. 26) that 1.0wt% of DMSO solvent residue was detected.

### Embodiment 9: solid-state stability experiment

In order to evaluate the solid stability of the free anhydrous crystal form B, an appropriate amount of the sample was respectively weighed, placed at 60°C for 24 hours, and exposed at 25°C/60% RH and 40°C/75% RH for one week. The crystal changes of the solid samples placed under different conditions were tested by XRPD, and the chemical stability was evaluated by HPLC. The characterization results are summarized in Table 13. The results showed that the HPLC purity of the crystal form B sample was not decreased obviously under the test conditions, and the crystal form was not changed.

**Table 13**

| Sample | Condition | Time | Purity (area%) | Relative to initial purity (%) | Crystal form variation |
|---|---|---|---|---|---|
| Free crystal form B | -- | Start | 99.91 | - | -- |
| | 25°C/60%RH/ exposure | One week | 99.90 | 99.99 | Constant |
| | 40°C/75%RH/ exposure | One week | 99.90 | 99.99 | Constant |
| | 60°C/closed | 24 hours | 99.91 | 100.00 | Constant |

In order to further study the transformation relationship between the free anhydrous crystal form and the hydrate crystal form, a suspension competition test was carried out on the anhydrous crystal form B/G and the hydrate crystal form A. Firstly, the suspension competition test between amorphous form B and G was set up at 5°C, room temperature and 50°C in ACN and room temperature in EtOAc. The specific steps are as follows: 1) saturated solution of the free state was prepared in different solvent systems at corresponding temperatures; 2) the corresponding free crystal form sample was added into 0.5 mL of the saturated solution to form a suspension; 3) the mixture was magnetically stirred under corresponding temperature conditions; 4) after stirring for 1 to 5 days, the solid was separated for XRPD testing. The results are shown in Table 14, and only the free crystal form B was obtained under the test conditions.

Therefore, a suspension competition test of stable anhydrous crystal form B at room temperature and hydrate form A with different water activities (a_{w}=0/0.2/0.4/0.6/0.8/1.0) of ACN/H₂O at room temperature was set up. The results are shown in Table 14, the anhydrous crystal form B was obtained under the condition of a_{w}=0 to 0.4, and the hydrate crystal form A was obtained in a_{w}=0.6 to 1 (pure water). The XRPD patterns of the solid obtained in the experiments are as shown in Figs. 27 to **Error! Reference source not found.**

**Table 14 Competition test results of different crystal suspension in free state**

| Test number (821726-) | Initial sample | Solvent (v:v) | Temperature | result |
|---|---|---|---|---|
| 22-B1 | Free state Crystal form B/G (mixing ratio 1:1) | ACN (a_{w} to 0) | 5°C | Free crystal form B |
| 22-B2 | | ACN (a_{w} to 0) | Room temperature | Free crystal form B |
| 22-B3 | | ACN (a_{w} to 0) | 50°C | Free crystal form B |
| 22-B4 | | EtOAc (a_{w} to 0) | Room temperature | Free crystal form B |
| 22-A1 | Free state Crystal form A/B (mixing ratio 1:1) | ACN (a_{w} to 0) | Room temperature | Free crystal form B |
| 22-A2 | | ACN/H₂O (981:19, a_{w} to 0.2) | | Free crystal form B |
| 22-A3 | | ACN/H₂O (957:43, a_{w} to 0.4) | | Free crystal form B |
| 22-A4 | | ACN/H₂O (924:76, a_{w} to 0.6) | | Free crystal form B |
| 22-A5 | | ACN/H₂O (87:13, a_{w} to 0.8) | | Free crystal form B |
| 22-A6 | | H₂O (a_{w} to 1.0) | | Free crystal form B |

It can be seen from the above table that the free anhydrous crystal form B can exist stably under certain water activity conditions, and the hydrate crystal form may have the risk of dehydration in the subsequent development, so the anhydrous crystal form B has better stability and subsequent druggability.

### Effect embodiment:

### I. Blocking activity of the compound of formula (I) on sodium channel 1.8 (NaV1.8)

1. Test method: the patch-clamp technique was used to detect the effects of compound on the voltage-gated sodium channels (NaV) 1.1 to 1.8 subtype currents
2. Preparation and analysis of administered preparation
2.1 Preparation method of storage solution of administered preparation

**Control:** appropriate volume of DMSO was weighed as storage solution.

**Test compound:** a suitable mass of the compound of formula (I) (actual quantity = theoretical concentration * volume × molecular weight/purity) was weighed, the required volume of DMSO was calculated according to the formula, and then the final required mass of DMSO was converted. The powder was then dissolved with the weighed DMSO. The actual concentration of the storage solution was calculated on the basis of the final DMSO usage, and generally the actual concentration of the storage solution was slightly different from the theoretical concentration.

### 2.2 Preparation method and concentration of working solution of administered preparation

Before the NaV channel current test, the storage solutions of the control and test compound were diluted into 10 mL extracellular fluid as the working solution, and ultrasonic treatment was performed for 20 minutes.

### 3. Experimental system

### 3. 1. Cell culture

1) the specific information of CHO cell line stably expressing NaV1.8 channel was as follows: SCN10A: NM_006514
2) Cells were cultured in HAM'S/F-12 medium containing 10% fetal bovine serum, 10 µg/mL Blasticidin, 200 µg/mL Hygromycin B and 100 µg/mL Zeocin at 37°C and 5% carbon dioxide concentration.
3) Cell passage: the old medium was removed and washed once with PBS, and then 1 mL of 0.25% - Trypsin-EDTA solution was added and incubated at 37°C for 1.5 minutes. When the cells were detached from the bottom of the plate, 5 mL of 37°C prewarmed complete medium was added. The cell suspension was gently pipetted to dissociate the aggregated cells. The cell suspension was transferred to a sterile centrifuge tube, and the cells were collected by centrifugation at 1000 rpm for 5 minutes. For expansion or maintenance culture, cells were seeded in 6 cm cell culture dishes, and the number of cells seeded in each cell culture dish was 2.5*10⁵ cells (final volume: 5 mL).

1) To maintain the electrophysiological activity of cells, the cell density must not exceed 80%.
2) For patch-clamp detection, cells were separated with 0.25%-Trypsin-EDTA before the experiment, seeded into a 24-well plate with a pre-placed coverslip at a density of 8*10³ cells per well (final volume: 500 µL), tetracycline was added, and it was tested the next day.

### 3.2. Electrophysiological solution

1) Extracellular fluid: 140 mM NaCl, 3.5mM KCl, 2mM CaCl₂, 10mM HEPES, 1.25mM NaH₂PO₄, 1mM MgCl₂, 10mM Glucose, pH=7.4 (NaOH).
2) Intracellular fluid: 50mM CsCl, 10mM NaCl, 10mM HEPES, 20mM EGTA, 60mM CsF, pH=7.2 (CsOH).

### 4. Test method

### 4.1. The instruments are as shown in the following Table 15

**Table 15: Instrument suppliers and models**

| Name | Supplier | Model |
|---|---|---|
| Amplifier | HEKA (Germany) | EPC10 |
| Microelectrode manipulator | Sutter Instruments (USA) | MP285 |
| Micropipette puller | Sutter Instruments (USA) | P97 |
| Microscope | Olympus (Japan) | IX71 |
| Fur glass tube | Sutter Instruments (USA) | BF150-86-10 |
| Data acquisition and analysis | HEKA (Germany) | PATCHMASTERPATCHMA |

### 4.2 Patch-clamp detection

The voltage stimulation solution of whole-cell patch clamp recording NaV channel current was as follows: firstly, the cell membrane potential was clamped to -130mV, and then the voltage was step to -40mV or -20mV for 8 seconds at a step interval of 10mv. The clamp voltage was maintained at-120mV and data acquisition was repeated every 20 seconds. The peak amplitude of the inward current was measured to determine its semi-inactive voltage.

The cell-clamp electrical potential was set at -120mV The resting and semi-inactive inhibition of sodium current was measured by double-pulse mode. Double-pulse mode was performed by two 0mV depolarization test pulses (TP1 and TP2) for 50ms. The conditional voltage between two depolarization pulses was set near the semi-inactive voltage (for 8s). Before a second depolarizing pulse was given, the cell membrane potential was clamped to -120 mV for 20 ms to allow compound unbinding, and the inactive channels were restored. Data acquisition was repeated at 20s intervals and the current peaks at two test pulses were measured.

The experimental data was acquired by the EPC-10 amplifier (HEKA) and stored in the PATCHMASTER (HEKA) software (software version: v2x73.2).

Capillary glass tubes (BF150-86-10, Sutter Instruments) were drawn into recording electrodes using a micropipette puller (P97, Sutter Instruments). The microelectrode manipulator (MP285) was manipulated under an inverted microscope (IX71) to touch the recording electrode to the cells, and a negative pressure suction was given to form a GQ seal. After the GQ seal was formed, rapid capacitance compensation was carried out, and then negative pressure was continuously applied to suck the cell membrane, forming a whole-cell recording mode. Then, the slow capacitor was compensated and the film capacitance and series resistance were recorded, without leakage compensation.

While the NaV channel current recorded in the whole cell was stable, the drug was administered, and each drug concentration was applied for 5 minutes (or the current is stabilized) and then the next concentration was detected; each test compound was detected at multiple concentrations. A cell-covered coverslip was placed in a recording bath in an inverted microscope, and the test compound and the external fluid without the compound flowed sequentially through the recording chamber from low concentration to high concentration using gravity perfusion method to act on the cell, and the fluid was exchanged using a vacuum pump in the recording. The current detected by each cell in the external fluid without the compounds was used as its own control group. Multiple cells were detected independently and repeatedly. All electrophysiological experiments were conducted at room temperature.

### 4.3 Data analysis

Firstly, the current after each drug concentration and the blank control current were standardized, and then the blocking rate corresponding to each drug concentration was calculated. The mean and standard error were calculated for each concentration, and all of the above values were calculated using Microsoft Excel 2013. In addition, the semi-inhibitory concentration of each compound was calculated by the following equation using the IGOR software: blocking rate = 1/[1+(IC₅₀/c)^{h}].

The dose-dependent effect was fitted nonlinearly by the above equation, wherein C represented the drug concentration, IC₅₀ was the semi-inhibitory concentration, and H represented the Hill coefficient. Curve fitting and IC₅₀ calculation were completed by the IGOR software (software version: 6.0.1.0).

In this embodiment, the half blocking activity (IC₅₀) of the compound of formula (I) for NaV1.8 was determined as shown in Table 16.:

**Table 16: IC₅₀ values of blocking activity of the compound of formula (I) for NaV1.8 (nM)**

| Numbering | NaV1.8 IC₅₀ (nM) |
|---|---|
| Compound of formula (I) | 6.2 |

The compound of formula (I) has an obvious blocking effect on NaV1.8 channel activity.

### II. Pharmacokinetic experiments of the compound of formula (I)

In this experimental embodiment, *in vivo* pharmacokinetic evaluation was evaluated in rats by single intravenous injection or intragastric oral administration.

Experimental methods and conditions: male Sprague Dawley rats; all animals were fasted overnight, and were given a single dose of the compound to be tested at 1 mg/kg (intravenous injection, solvent 5% DMSO/10% Solutol/85% Saline) and 10 mg/kg (intragastric administration) respectively, at 5 min, 15 min, 30 min, 1 hr, 2 hr, 4 hr, 6 hr, 8 hr and 24 h after drug administration, blood was collected from the submandibular vein, about 0.20 mL per sample was collected, heparin sodium was used for anticoagulation, and then it was placed on ice after collection and centrifuged within 1 hour to separate the plasma for testing. The concentration of the drug in the plasma was measured by liquid tandem mass spectrometry (LC/MS/MS), and the measured concentration was used to calculate pharmacokinetic parameters. The results are shown in following Table 17 and Table 18:

**Table 17: Pharmacokinetics of intravenous administration (1 mg/kg)**

| Numbering | T_{1/2} (hr) | AUC_{inf} (ng*hr/mL) | V_{Z} (mL/kg) | CL (mL/min/kg) |
|---|---|---|---|---|
| Compound of formula (I) | 0.70 | 1462.27 | 699.19 | 11.44 |

**Table 18: Pharmacokinetics of intragastric injection administration (10 mg/kg)**

| Numbering | T_{1/2} (hr) | Cₘₐₓ (ng/mL) | AUC_{inf} (ng*hr/mL) | F (%) |
|---|---|---|---|---|
| Compound of formula (I) | 2.51 | 1326.67 | 10614.74 | 72.59 |

The compound of formula (I) has good pharmacokinetic absorption in rats and has a pharmacokinetic advantage.

## Claims

1. A crystal form A of a compound represented by formula (I), 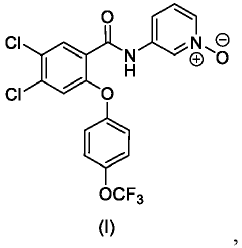 wherein, the X-ray powder diffraction pattern of the crystal form A comprises characteristic diffraction peaks at the following angles of 2θ: 16.63±0.2°, 18.04±0.2°, 20.59±0.2°, 23.38±0.2°, 23.96±0.2°, and 29.19±0.2°.

2. The crystal form A according to claim 1, wherein, the X-ray powder diffraction pattern of the crystal form A comprises characteristic diffraction peaks at the following angles of 2θ: 12.46±0.2°, 13.11±0.2°, 16.63±0.2°, 18.04±0.2°, 20.59±0.2 °, 23.38±0.2°, 23.96±0.2°, 27.66±0.2°, 29.19±0.2° and 29.82±0.2°.

3. The crystal form A according to claim 2, wherein, the X-ray powder diffraction pattern of the crystal form A has an X-ray powder diffraction pattern substantially as shown in Fig. 1.

4. The crystal form A according to claim 3, wherein, the crystal form A is a hydrate, and the moisture content of the hydrate is 3.0 to 5.0wt%.

5. A crystal form B of the compound represented by formula (I), wherein, the X-ray powder diffraction pattern of the crystal form B comprises characteristic diffraction peaks at the following angles of 2θ: 12.28±0.2°, 14.47±±0.2°, 18.86±0.2°, 23.09±0.2°, 25.50±0.2°, and 27.58±0.2°.

6. The crystal form B according to claim 5, wherein, the X-ray powder diffraction pattern of the crystal form B comprises characteristic diffraction peaks at the following angles of 2θ: 12.28±0.2°, 14.47±0.2°, 16.81±0.2°, 18.86±0.2°, 19.78±0.2 °, 23.09±0.2°, 25.09±0.2°, 25.50±0.2°, 27.58±0.2° and 28.19±0.2°.

7. The crystal form B according to claim 6, wherein, the X-ray powder diffraction pattern of the crystal form B has an X-ray powder diffraction pattern substantially as shown in Fig. 4.

8. A crystal form C of the compound represented by formula (I), wherein, the X-ray powder diffraction pattern of the crystal form C comprises characteristic diffraction peaks at the following angles of 2θ: 8.22±0.2°, 17.33±±0.2°, 19.55±±0.2°, 20.27±±0.2°, 21.99±0.2°, and 24.90±0.2°.

9. The crystal form C according to claim 8, wherein, the X-ray powder diffraction pattern of the crystal form C comprises characteristic diffraction peaks at the following angles of 2θ: 8.22±0.2°, 13.80±0.2°, 17.33±0.2°, 19.55±0.2°, 20.27±0.2 °, 21.99±0.2°, 23.00±0.2°, 23.95±0.2°, 24.90±0.2° and 26.10±0.2°.

10. The crystal form C according to claim 9, wherein, the X-ray powder diffraction pattern of the crystal form C has an X-ray powder diffraction pattern substantially as shown in Fig. 7.

11. The crystal form C according to claim 10, wherein, the crystal form C is a 1,4-dioxane solvate, and the content of the 1,4-dioxane is 3wt% to 17wt%.

12. A crystal form D of the compound represented by formula (I), wherein, the X-ray powder diffraction pattern of the crystal form D comprises characteristic diffraction peaks at the following angles of 2θ: 5.63±0.2°, 16.81±0.2°, 20.40±0.2°, 21.50±0.2°, 22.23±0.2°, and 26.08±0.2°.

13. The crystal form D according to claim 12, wherein, the X-ray powder diffraction pattern of the crystal form D comprises characteristic diffraction peaks at the following angles of 2θ: 5.63±0.2°, 11.02±0.2°, 16.81±0.2°, 19.58±0.2°, 20.40±0.2 °, 21.50±0.2°, 22.23±0.2°, 24.17±0.2°, 26.08±0.2° and 28.44±0.2°.

14. The crystal form D according to claim 13, wherein, the X-ray powder diffraction pattern of the crystal form D has an X-ray powder diffraction pattern substantially as shown in Fig. 10.

15. The crystal form D according to claim 14, wherein, the crystal form D is a methyl ethyl ketone solvate, and the content of the methyl ethyl ketone is 4wt% to 14wt%.

16. A crystal form E of the compound represented by formula (I), wherein, the X-ray powder diffraction pattern of the crystal form E comprises characteristic diffraction peaks at the following angles of 2θ: 5.75±0.2°, 13.71±0.2°, 18.29±0.2°, 20.18±0.2°, 22.92±0.2°, and 23.96±0.2°.

17. The crystal form E according to claim 16, wherein, the X-ray powder diffraction pattern of the crystal form E comprises characteristic diffraction peaks at the following angles of 2θ: 5.75±0.2°, 13.71±0.2°, 16.65±0.2°, 17.17±0.2°, 18.29±0.2 °, 20.18±0.2°, 22.92±0.2°, 23.96±0.2°, 24.76±0.2° and 29.18±0.2°.

18. The crystal form E according to claim 17, wherein, the X-ray powder diffraction pattern of the crystal form E has an X-ray powder diffraction pattern substantially as shown in Fig. 13.

19. The crystal form E according to claim 18, wherein, the crystal form E is a tetrahydrofuran solvate, and the content of the tetrahydrofuran is 2wt% to 14wt%.

20. A crystal form F of the compound represented by formula (I), wherein, the X-ray powder diffraction pattern of the crystal form F comprises characteristic diffraction peaks at the following angles of 2θ: 17.24±0.2°, 20.28±0.2°, 23.03±0.2°, 23.96±0.2°, 24.89±0.2°, and 28.96±0.2°.

21. The crystal form F according to claim 20, wherein, the X-ray powder diffraction pattern of the crystal form F comprises characteristic diffraction peaks at the following angles of 2θ: 5.78±0.2°, 14.31±0.2°, 17.24±0.2°, 20.28±0.2°, 22.06±0.2 °, 23.03±0.2°, 23.96±0.2°, 24.89±0.2°, 26.27±0.2° and 28.96±0.2°.

22. The crystal form F according to claim 21, wherein, the X-ray powder diffraction pattern of the crystal form F has an X-ray powder diffraction pattern substantially as shown in Fig. 16.

23. The crystal form F according to claim 22, wherein, the crystal form F is a chloroform solvate, and the content of the chloroform is 5wt% to 21wt%.

24. A crystal form G of the compound represented by formula (I), wherein, the X-ray powder diffraction pattern of the crystal form G comprises characteristic diffraction peaks at the following angles of 2θ: 15.53±0.2°, 17.08±0.2°, 21.41±0.2°, 23.23±0.2°, 26.00±0.2°, and 28.49±0.2°.

25. The crystal form G according to claim 24, wherein, the X-ray powder diffraction pattern of the crystal form G comprises characteristic diffraction peaks at the following angles of 2θ: 10.54±0.2°, 13.02±0.2°, 15.53±0.2°, 17.08±0.2°, 21.41±0.2 °, 23.23±0.2°, 25.10±0.2°, 26.00±0.2°, 27.17±0.2° and 28.49±0.2°.

26. The crystal form G according to claim 25, wherein, the X-ray powder diffraction pattern of the crystal form G has an X-ray powder diffraction pattern substantially as shown in Fig. 19.

27. A pharmaceutical composition, comprising the crystal form A according to any one of claims 1 to 4, the crystal form B according to any one of claims 5 to 7, the crystal form C according to any one of claims 8 to 11, the crystal form D according to any one of claims 12 to 15, the crystal form E according to any one of claims 16 to 19, the crystal form F according to any one of claims 20 to 23, or the crystal form G according to any one of claims 24 to 26.

28. The pharmaceutical composition according to claim 27, wherein, the pharmaceutical composition further comprises a pharmaceutically acceptable carrier, excipient, diluent, adjuvant, vehicle or their combination.

29. Use of the crystal form A according to any one of claims 1 to 4 or the crystal form B according to any one of claims 5 to 7, the crystal form C according to any one of claims 8 to 11, the crystal form D according to any one of claims 12 to 15, the crystal form E according to any one of claims 16 to 19, the crystal form F according to any one of claims 20 to 23, the crystal form G according to any one of claims 24 to 26, or the pharmaceutical composition according to claim 27 or 28 in the manufacture of a medicament for inhibiting a voltage-gated sodium channel of an individual.

30. The use according to claim 29, wherein, the voltage-gated sodium channel is NaVL.8.

31. Use of the crystal form A according to any one of claims 1 to 4, the crystal form B according to any one of claims 5 to 7, the crystal form C according to any one of claims 8 to 11, the crystal form D according to any one of claims 12 to 15, the crystal form E according to any one of claims 16 to 19, the crystal form F according to any one of claims 20 to 23, the crystal form G according to any one of claims 24 to 26, or the pharmaceutical composition according to claim 27 or 28 in the manufacture of a medicament for treating and/or preventing pain, cough or alleviating their severity of an individual.

32. The use according to claim 31, wherein, the pain is selected from chronic pain, bowel pain, neuropathic pain, musculoskeletal pain, acute pain, inflammatory pain, cancer pain, primary pain, postoperative pain, visceral pain, multiple sclerosis, Charcot-Marie-Tooth syndrome, incontinence and arrhythmia.
